# EUROPEAN PATENT APPLICATION

(11) **EP 2 392 585 A2**
(43) Date of publication of application: **07.12.2011**
(21) Application number: 11006391.4
(22) Date of filing: 17.10.2007
(51) Int. Cl.: C07K 7/08, C07K 14/00, C12N 9/02, A61K 38/44

(54) **Scaffold proteins for recombinant peptide aptamers**

(62) Divisional of application: 07020336.9
(71) Applicant: Chemotherapeutisches Forschungsinstitut Georg-Speyer-Haus, 60596 Frankfurt (DE)
(72) Inventor: Groner, Bernd, Prof. Dr., 60322 Frankfurt am Main (DE); Borghouts-Heinz, Corina, Dr., 55278 Dalheim (DE)
(74) Representative: Graf von Stosch, Andreas

(57) **Abstract**

The present invention relates to scaffold proteins for recombinant peptide aptamers, especially to a modified variant of human thioredoxin, and to the aptamers themselves, especially apatamers binding to the transcription factor Stat3. The invention furthermore relates to Fusion Proteins of the scaffold protein and the aptamer, nucleic acids coding for these peptides or proteins, medicaments comprising same and their uses, especially in cancer therapy.

SEQUENCE LISTING

<110>  Chemotherapeutisches Forschungsinstitut Georg-Speyer-Haus

<120>  Scaffold proteins for recombinant peptide aptamers

<130>  GS02P002EPT1

<140>  EP 07020336.9
<141>  2007-10-17

<160>  54

<170>  PatentIn version 3.3

<210>  1
<211>  112
<212>  PRT
<213>  Unknown

<220>
<223>  Amino acid sequence of the unmodified bacterial thioredoxin
       (wild-type; wt-bTrx)

<400>  1

Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Cys Gly Pro Cys Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Thr Thr Lys Val Gly Ala Leu Ser
                85                  90                  95

Lys Gly Gln Leu Lys Glu Phe Leu Asp Ala Asn Leu Ala Glu Phe Gly
            100                 105                 110

<210>  2
<211>  112
<212>  PRT
<213>  Artificial

<220>
<223>  Amino acid sequence of a modified bacterial thioredoxin
       (bTrx-Dcys2)

<400>  2

Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Ser Gly Pro Ser Lys Met Ile Ala Pro Ile Leu Asp Glu Ile Ala Asp
        35                  40                  45

Glu Tyr Gln Gly Lys Leu Thr Val Ala Lys Leu Asn Ile Asp Gln Asn
    50                  55                  60

Pro Gly Thr Ala Pro Lys Tyr Gly Ile Arg Gly Ile Pro Thr Leu Leu
65                  70                  75                  80

Leu Phe Lys Asn Gly Glu Val Ala Thr Thr Lys Val Gly Ala Leu Ser
                85                  90                  95

Lys Gly Gln Leu Lys Glu Phe Leu Asp Ala Asn Leu Ala Glu Phe Gly
            100                 105                 110

<210>  3
<211>  105
<212>  PRT
<213>  Homo sapiens

<220>
<223>  Amino acid sequence of the unmodified human thioredoxin
       (wild-type; wt-hTrx)

<400>  3

Met Val Lys Gln Ile Glu Ser Lys Thr Ala Phe Gln Glu Ala Leu Asp
1               5                   10                  15

Ala Ala Gly Asp Lys Leu Val Val Val Asp Phe Ser Ala Thr Trp Cys
            20                  25                  30

Gly Pro Cys Lys Met Ile Lys Pro Phe Phe His Ser Leu Ser Glu Lys
        35                  40                  45

Tyr Ser Asn Val Ile Phe Leu Glu Val Asp Val Asp Asp Cys Gln Asp
    50                  55                  60

Val Ala Ser Glu Cys Glu Val Lys Cys Met Pro Thr Phe Gln Phe Phe
65                  70                  75                  80

Lys Lys Gly Gln Lys Val Gly Glu Phe Ser Gly Ala Asn Lys Glu Lys
                85                  90                  95

Leu Glu Ala Thr Ile Asn Glu Leu Val
            100                 105

<210>  4
<211>  105
<212>  PRT
<213>  Artificial

<220>

<223>  Amino acid sequence of a modified human thioredoxin (hTrx-delta-cys2)

<400>  4

Met Val Lys Gln Ile Glu Ser Lys Thr Ala Phe Gln Glu Ala Leu Asp
1               5                   10                  15

Ala Ala Gly Asp Lys Leu Val Val Val Asp Phe Ser Ala Thr Trp Gly
            20                  25                  30

Gly Pro Gly Lys Met Ile Lys Pro Phe Phe His Ser Leu Ser Glu Lys
        35                  40                  45

Tyr Ser Asn Val Ile Phe Leu Glu Val Asp Val Asp Asp Cys Gln Asp
    50                  55                  60

Val Ala Ser Glu Cys Glu Val Lys Cys Met Pro Thr Phe Gln Phe Phe
65                  70                  75                  80

Lys Lys Gly Gln Lys Val Gly Glu Phe Ser Gly Ala Asn Lys Glu Lys
                85                  90                  95

Leu Glu Ala Thr Ile Asn Glu Leu Val
            100                 105

<210>  5
<211>  105
<212>  PRT
<213>  Artificial

<220>
<223>  Amino acid sequence of a modified human thioredoxin (hTrx-delta-cys5)

<400>  5

Met Val Lys Gln Ile Glu Ser Lys Thr Ala Phe Gln Glu Ala Leu Asp
1               5                   10                  15

Ala Ala Gly Asp Lys Leu Val Val Val Asp Phe Ser Ala Thr Trp Gly
            20                  25                  30

Gly Pro Gly Lys Met Ile Lys Pro Phe Phe His Ser Leu Ser Glu Lys
        35                  40                  45

Tyr Ser Asn Val Ile Phe Leu Glu Val Asp Val Asp Asp Ser Gln Asp
    50                  55                  60

Val Ala Ser Glu Ser Glu Val Lys Ser Met Pro Thr Phe Gln Phe Phe
65                  70                  75                  80

Lys Lys Gly Gln Lys Val Gly Glu Phe Ser Gly Ala Asn Lys Glu Lys
                85                  90                  95

Leu Glu Ala Thr Ile Asn Glu Leu Val
            100                 105

<210>  6
<211>  43
<212>  PRT
<213>  Artificial

<220>
<223>  Amino acid sequence of the modified human aptamer DD3 binding to
       the dimerization domain of the transcription factor Stat3
       (DD3-delta-cys)

<400>  6

Ser Pro Ile Ser Ile Pro Ile Gly Phe Val Val Arg His Ser Ala Leu
1               5                   10                  15

His Met Ala Val Gly Pro Leu Ser Trp Pro Ala Arg Val Ser Gly Tyr
            20                  25                  30

Ser Phe Ala Leu Glu Val Leu Thr Asn Phe Gly
        35                  40

<210>  7
<211>  43
<212>  PRT
<213>  Homo sapiens

<220>
<223>  Amino acid sequence of the human aptamer DD3 binding to the
       dimerization domain of the transcription factor Stat3 (DD3-wild
       type)

<400>  7

Ser Pro Ile Ser Ile Pro Ile Gly Phe Val Val Arg His Cys Ala Leu
1               5                   10                  15

His Met Ala Val Gly Pro Leu Ser Trp Pro Ala Arg Val Ser Gly Tyr
            20                  25                  30

Ser Phe Ala Leu Glu Val Leu Thr Asn Phe Gly
        35                  40

<210>  8
<211>  20
<212>  PRT
<213>  Homo sapiens

<220>
<223>  Amino acid sequence of the human aptamer DD3 binding to the
       dimerization domain of the transcription factor Stat3 (DD3-1.10)

<400>  8

Ser Pro Ile Ser Ile Pro Ile Gly Phe Val Val Arg His Cys Ala Leu
1               5                   10                  15

His Met Ala Gly
            20

<210>  9

<211>  20
<212>  PRT
<213>  Artificial

<220>
<223>  Shortened modified amino acid sequence of the human aptamer DD3
       binding to the dimerization domain of the transcription factor
       Stat3 (DD3-1.10-delta-cys)

<400>  9

Ser Pro Ile Ser Ile Pro Ile Gly Phe Val Val Arg His Ser Ala Leu
1               5                   10                  15

His Met Ala Gly
            20

<210>  10
<211>  21
<212>  PRT
<213>  Homo sapiens

<220>
<223>  Shortened amino acid sequence of the human aptamer DD3 binding to
       the dimerization domain of the transcription factor Stat3
       (DD3-3.8)

<400>  10

Val Arg His Cys Ala Leu His Met Ala Val Gly Pro Leu Ser Trp Pro
1               5                   10                  15

Ala Arg Val Ser Gly
            20

<210>  11
<211>  21
<212>  PRT
<213>  Artificial

<220>
<223>  Shortened modified amino acid sequence of the human aptamer DD3
       binding to the dimerization domain of the transcription factor
       Stat3 (DD3-3.8-delta-cys)

<400>  11

Val Arg His Ser Ala Leu His Met Ala Val Gly Pro Leu Ser Trp Pro
1               5                   10                  15

Ala Arg Val Ser Gly
            20

<210>  12
<211>  28
<212>  PRT
<213>  Homo sapiens

<220>
<223>  Shortened amino acid sequence of the human aptamer DD3 binding to
       the dimerization domain of the transcription factor Stat3
       (DD3-4.6)

<400>  12

Leu His Met Ala Val Gly Pro Leu Ser Trp Pro Ala Arg Val Ser Gly
1               5                   10                  15

Tyr Ser Phe Ala Leu Glu Val Leu Thr Asn Phe Gly
            20                  25

<210>  13
<211>  21
<212>  PRT
<213>  Homo sapiens

<220>
<223>  Shortened amino acid sequence of the human aptamer DD3 binding to
       the dimerization domain of the transcription factor Stat3
       (DD3-5.6)

<400>  13

Leu Ser Trp Pro Ala Arg Val Ser Gly Tyr Ser Phe Ala Leu Glu Val
1               5                   10                  15

Leu Thr Asn Phe Gly
            20

<210>  14
<211>  116
<212>  PRT
<213>  Artificial

<220>
<223>  Amino acid sequence of a Fusion Protein of an aptamer (Y2..Y4) in
       a modified bacterial thioredoxin (bTrx-delta-cys2)

<220>
<221>  SITE
<222>  (35)..(36)
<223>  site for insertion of an aptamer (Y2..Y4) in a modified bacterial
       thioredoxin between residues 35 and 36

<400>  14

Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Ser Gly Pro Pro Gly Gly Pro Ser Lys Met Ile Ala Pro Ile Leu Asp
        35                  40                  45

Glu Ile Ala Asp Glu Tyr Gln Gly Lys Leu Thr Val Ala Lys Leu Asn
    50                  55                  60

Ile Asp Gln Asn Pro Gly Thr Ala Pro Lys Tyr Gly Ile Arg Gly Ile
65                  70                  75                  80

Pro Thr Leu Leu Leu Phe Lys Asn Gly Glu Val Ala Thr Thr Lys Val

85                  90                  95

Gly Ala Leu Ser Lys Gly Gln Leu Lys Glu Phe Leu Asp Ala Asn Leu
            100                 105                 110

Ala Glu Phe Gly
        115

<210>  15
<211>  109
<212>  PRT
<213>  Artificial

<220>
<223>  Amino acid sequence of a Fusion Protein of an aptamer (Y2..Y4) in
       a modified human thioredoxin (hTrx-delta-cys2)

<220>
<221>  SITE
<222>  (34)..(35)
<223>  site for insertion of an aptamer ((Y2..Y4) in a modified human
       thioredoxin between residues 34 and 35

<400>  15

Met Val Lys Gln Ile Glu Ser Lys Thr Ala Phe Gln Glu Ala Leu Asp
1               5                   10                  15

Ala Ala Gly Asp Lys Leu Val Val Val Asp Phe Ser Ala Thr Trp Gly
            20                  25                  30

Gly Pro Pro Gly Gly Pro Gly Lys Met Ile Lys Pro Phe Phe His Ser
        35                  40                  45

Leu Ser Glu Lys Tyr Ser Asn Val Ile Phe Leu Glu Val Asp Val Asp
    50                  55                  60

Asp Cys Gln Asp Val Ala Ser Glu Cys Glu Val Lys Cys Met Pro Thr
65                  70                  75                  80

Phe Gln Phe Phe Lys Lys Gly Gln Lys Val Gly Glu Phe Ser Gly Ala
                85                  90                  95

Asn Lys Glu Lys Leu Glu Ala Thr Ile Asn Glu Leu Val
            100                 105

<210>  16
<211>  109
<212>  PRT
<213>  Artificial

<220>
<223>  Amino acid sequence of a Fusion Protein of an aptamer (Y2..Y4) in
       a modified human thioredoxin (hTrx-delta-cys5)

<220>
<221>  SITE

<222>  (34)..(35)
<223>  site for insertion of an aptamer (Y2..Y4) in a mmodified human
       thioredoxin between residues 34 and 35

<400>  16

Met Val Lys Gln Ile Glu Ser Lys Thr Ala Phe Gln Glu Ala Leu Asp
1               5                   10                  15

Ala Ala Gly Asp Lys Leu Val Val Val Asp Phe Ser Ala Thr Trp Gly
            20                  25                  30

Gly Pro Pro Gly Gly Pro Gly Lys Met Ile Lys Pro Phe Phe His Ser
        35                  40                  45

Leu Ser Glu Lys Tyr Ser Asn Val Ile Phe Leu Glu Val Asp Val Asp
    50                  55                  60

Asp Ser Gln Asp Val Ala Ser Glu Ser Glu Val Lys Ser Met Pro Thr
65                  70                  75                  80

Phe Gln Phe Phe Lys Lys Gly Gln Lys Val Gly Glu Phe Ser Gly Ala
                85                  90                  95

Asn Lys Glu Lys Leu Glu Ala Thr Ile Asn Glu Leu Val
            100                 105

<210>  17
<211>  129
<212>  PRT
<213>  Artificial

<220>
<223>  Amino acid sequence of a Fusion Protein of the shortened aptamer
       DD3-1.10 in a modified human thioredoxin (hTrx-delta-cys5)

<400>  17

Met Val Lys Gln Ile Glu Ser Lys Thr Ala Phe Gln Glu Ala Leu Asp
1               5                   10                  15

Ala Ala Gly Asp Lys Leu Val Val Val Asp Phe Ser Ala Thr Trp Gly
            20                  25                  30

Gly Pro Ser Pro Ile Ser Ile Pro Ile Gly Phe Val Val Arg His Cys
        35                  40                  45

Ala Leu His Met Ala Gly Pro Gly Gly Pro Gly Lys Met Ile Lys Pro
    50                  55                  60

Phe Phe His Ser Leu Ser Glu Lys Tyr Ser Asn Val Ile Phe Leu Glu
65                  70                  75                  80

Val Asp Val Asp Asp Ser Gln Asp Val Ala Ser Glu Ser Glu Val Lys
                85                  90                  95

Ser Met Pro Thr Phe Gln Phe Phe Lys Lys Gly Gln Lys Val Gly Glu
            100                 105                 110

Phe Ser Gly Ala Asn Lys Glu Lys Leu Glu Ala Thr Ile Asn Glu Leu
        115                 120                 125

Val

<210>  18
<211>  129
<212>  PRT
<213>  Artificial

<220>
<223>  Amino acid sequence of a Fusion Protein of the shortened and
       modified aptamer DD3-1.10-delta-cys in a modified human
       thioredoxin(hTrx-delta-cys5)

<400>  18

Met Val Lys Gln Ile Glu Ser Lys Thr Ala Phe Gln Glu Ala Leu Asp
1               5                   10                  15

Ala Ala Gly Asp Lys Leu Val Val Val Asp Phe Ser Ala Thr Trp Gly
            20                  25                  30

Gly Pro Ser Pro Ile Ser Ile Pro Ile Gly Phe Val Val Arg His Ser
        35                  40                  45

Ala Leu His Met Ala Gly Pro Gly Gly Pro Gly Lys Met Ile Lys Pro
    50                  55                  60

Phe Phe His Ser Leu Ser Glu Lys Tyr Ser Asn Val Ile Phe Leu Glu
65                  70                  75                  80

Val Asp Val Asp Asp Ser Gln Asp Val Ala Ser Glu Ser Glu Val Lys
                85                  90                  95

Ser Met Pro Thr Phe Gln Phe Phe Lys Lys Gly Gln Lys Val Gly Glu
            100                 105                 110

Phe Ser Gly Ala Asn Lys Glu Lys Leu Glu Ala Thr Ile Asn Glu Leu
        115                 120                 125

Val

<210>  19
<211>  131
<212>  PRT
<213>  Artificial

<220>
<223>  Amino acid sequence of a Fusion Protein of the shortened aptamer
       DD3-3.8 in a modified human thioredoxin (hTrx-delta-cys5)

<400>  19

Met Val Lys Gln Ile Glu Ser Lys Thr Ala Phe Gln Glu Ala Leu Asp
1               5                   10                  15

Ala Ala Gly Asp Lys Leu Val Val Val Asp Phe Ser Ala Thr Trp Gly
            20                  25                  30

Gly Pro Ser Val Arg His Cys Ala Leu His Met Ala Val Gly Pro Leu
        35                  40                  45

Ser Trp Pro Ala Arg Val Ser Gly Pro Gly Gly Pro Gly Lys Met Ile
    50                  55                  60

Lys Pro Phe Phe His Ser Leu Ser Glu Lys Tyr Ser Asn Val Ile Phe
65                  70                  75                  80

Leu Glu Val Asp Val Asp Asp Ser Gln Asp Val Ala Ser Glu Ser Glu
                85                  90                  95

Val Lys Ser Met Pro Thr Phe Gln Phe Phe Lys Lys Gly Gln Lys Val
            100                 105                 110

Gly Glu Phe Ser Gly Ala Asn Lys Glu Lys Leu Glu Ala Thr Ile Asn
        115                 120                 125

Glu Leu Val
    130

<210>  20
<211>  131
<212>  PRT
<213>  Artificial

<220>
<223>  Amino acid sequence of a Fusion Protein of the shortened and
       modified aptamer DD3-3.8-delta-cys in a modified human thioredoxin
       (hTrx-delta-cys5)

<400>  20

Met Val Lys Gln Ile Glu Ser Lys Thr Ala Phe Gln Glu Ala Leu Asp
1               5                   10                  15

Ala Ala Gly Asp Lys Leu Val Val Val Asp Phe Ser Ala Thr Trp Gly
            20                  25                  30

Gly Pro Ser Val Arg His Ser Ala Leu His Met Ala Val Gly Pro Leu
        35                  40                  45

Ser Trp Pro Ala Arg Val Ser Gly Pro Gly Gly Pro Gly Lys Met Ile
    50                  55                  60

Lys Pro Phe Phe His Ser Leu Ser Glu Lys Tyr Ser Asn Val Ile Phe
65                  70                  75                  80

Leu Glu Val Asp Val Asp Asp Ser Gln Asp Val Ala Ser Glu Ser Glu
                85                  90                  95

Val Lys Ser Met Pro Thr Phe Gln Phe Phe Lys Lys Gly Gln Lys Val
            100                 105                 110

Gly Glu Phe Ser Gly Ala Asn Lys Glu Lys Leu Glu Ala Thr Ile Asn
        115                 120                 125

Glu Leu Val
    130

<210>  21
<211>  138
<212>  PRT
<213>  Artificial

<220>
<223>  Amino acid sequence of a Fusion Protein of the shortened aptamer
       DD3-4.6 in a modified human thioredoxin (hTrx-delta-cys5)

<400>  21

Met Val Lys Gln Ile Glu Ser Lys Thr Ala Phe Gln Glu Ala Leu Asp
1               5                   10                  15

Ala Ala Gly Asp Lys Leu Val Val Val Asp Phe Ser Ala Thr Trp Gly
            20                  25                  30

Gly Pro Ser Leu His Met Ala Val Gly Pro Leu Ser Trp Pro Ala Arg
        35                  40                  45

Val Ser Gly Tyr Ser Phe Ala Leu Glu Val Leu Thr Asn Phe Gly Pro
    50                  55                  60

Gly Gly Pro Gly Lys Met Ile Lys Pro Phe Phe His Ser Leu Ser Glu
65                  70                  75                  80

Lys Tyr Ser Asn Val Ile Phe Leu Glu Val Asp Val Asp Asp Ser Gln
                85                  90                  95

Asp Val Ala Ser Glu Ser Glu Val Lys Ser Met Pro Thr Phe Gln Phe
            100                 105                 110

Phe Lys Lys Gly Gln Lys Val Gly Glu Phe Ser Gly Ala Asn Lys Glu
        115                 120                 125

Lys Leu Glu Ala Thr Ile Asn Glu Leu Val
    130                 135

<210>  22
<211>  131
<212>  PRT
<213>  Artificial

<220>
<223>  Amino acid sequence of a Fusion Protein of the shortened aptamer
      DD3-5.6 in a modified human thioredoxin (hTrx-delta-cys5)

<400>  22

Met Val Lys Gln Ile Glu Ser Lys Thr Ala Phe Gln Glu Ala Leu Asp
1               5                   10                  15

Ala Ala Gly Asp Lys Leu Val Val Val Asp Phe Ser Ala Thr Trp Gly
            20                  25                  30

Gly Pro Ser Leu Ser Trp Pro Ala Arg Val Ser Gly Tyr Ser Phe Ala
        35                  40                  45

Leu Glu Val Leu Thr Asn Phe Gly Pro Gly Gly Pro Gly Lys Met Ile
    50                  55                  60

Lys Pro Phe Phe His Ser Leu Ser Glu Lys Tyr Ser Asn Val Ile Phe
65                  70                  75                  80

Leu Glu Val Asp Val Asp Asp Ser Gln Asp Val Ala Ser Glu Ser Glu
                85                  90                  95

Val Lys Ser Met Pro Thr Phe Gln Phe Phe Lys Lys Gly Gln Lys Val
            100                 105                 110

Gly Glu Phe Ser Gly Ala Asn Lys Glu Lys Leu Glu Ala Thr Ile Asn
        115                 120                 125

Glu Leu Val
    130


<210>  23
<211>  336
<212>  DNA
<213>  Artificial

<220>
<223>  DNA sequence of a modified bacterial thioredoxin (bTrx-delta-cys2;
       coding for SEQ.ID.No.2)

<400>  23
atgagcgata aaattattca cctgactgac gacagttttg acacggatgt actcaaagcg      60

gacggggcga tcctcgtcga tttctgggca gagtggagcg gtccgagcaa aatgatcgcc     120

ccgattctgg atgaaatcgc tgacgaatat cagggcaaac tgaccgttgc aaaactgaac     180

atcgatcaaa accctggcac tgcgccgaaa tatggcatcc gtggtatccc gactctgctg     240

ctgttcaaaa acggtgaagt ggcgacaacc aaagtgggtg cactgtctaa aggtcagttg     300

aaagagttcc tcgacgctaa cctggccgaa ttcggg                               336


<210>  24
<211>  315
<212>  DNA
<213>  Artificial

<220>
<223>  DNA sequence of a modified human thioredoxin (hTrx-delta-cys5; coding
       for SEQ.ID.No.5)

<400>  24
atggtgaagc agatcgagag caagactgct tttcaggaag ccttggacgc tgcaggtgat      60

aaacttgtag tagttgactt ctcagccacg tggggcggtc cgggcaaaat gatcaagcct     120

ttctttcatt ccctctctga aaagtattcc aacgtgatat tccttgaagt agatgtggat     180

gactctcagg atgttgcttc agagtctgaa gtcaaaagca tgccaacatt ccagtttttt     240

aagaagggac aaaaggtggg tgaattttct ggagccaata aggaaaagct tgaagccacc     300

attaatgaat tcgtc                                                      315


<210>  25
<211>  129
<212>  DNA
<213>  Artificial

<220>
<223>  DNA sequence of the modified human aptamer DD3 binding to the
       dimerization domain of the transcription factor Stat3 (DD3-delta-cys;
       coding for SEQ.ID.No. 6)

<400>  25
tctcctatta gtattccgat tgggtttgtt gttcggcatt ctgcgcttca tatggcggtg      60

ggtccgttgt cttggccggc gagggtttct gggtattcgt ttgctttgga ggtgctgact     120

aatttcggt                                                             129


<210>  26
<211>  57
<212>  DNA
<213>  Homo sapiens

<220>
<223>  DNA sequence of the shortened human aptamer DD3 binding to the
       dimerization domain of the transcription factor Stat3 (DD3-1.10;
       coding for SEQ.ID.No. 8)

<400>  26
tctcctatta gtattccgat tgggtttgtt gttcggcatt gtgcgcttca tatggcc         57


<210>  27
<211>  57
<212>  DNA
<213>  Artificial

<220>
<223>  DNA sequence of the shortened modified human aptamer DD3 binding
       to the dimerization domain of the transcription factor Stat3
       (DD3-1.10-delta-cys; coding for SEQ.ID.No. 9)

<400>  27
tctcctatta gtattccgat tgggtttgtt gttcggcatt ctgcgcttca tatggcc         57


<210>  28
<211>  63
<212>  DNA
<213>  Homo sapiens

<220>
<223>  DNA sequence of the shortened human aptamer DD3-3.8 binding to
       the dimerization domain of the transcription factor Stat3
       (DD3-3.8; coding for SEQ.ID.No. 10)

<400>  28
gttcggcatt gtgcgcttca tatggcggtg ggtccgttgt cttggccggc gagggtttct      60

ggc                                                                    63


<210>  29
<211>  63
<212>  DNA
<213>  Artificial

<220>
<223>  DNA sequence of the shortened modified human aptamer DD3-3.8
       binding to the dimerization domain of the transcription factor
       Stat3 (DD3-3.8-delta-cys; coding for SEQ.ID.No. 11)

<400>  29
gttcggcatt ctgcgcttca tatggcggtg ggtccgttgt cttggccggc gagggtttct      60

ggc                                                                    63


<210>  30
<211>  396
<212>  DNA
<213>  Artificial

<220>
<223>  DNA sequence of a fusion protein between modified human
       thioredoxin (hTrx-delta-cys5) and the shortened aptamer DD3-3.8
       together with introduced restiction sites

<400>  30
atggtgaagc agatcgagag caagactgct tttcaggaag ccttggacgc tgcaggtgat      60

aaacttgtag tagttgactt ctcagccacg tggggcggtc cgggatccgt tcggcattgt     120

gcgcttcata tggcggtggg tccgttgtct tggccggcga gggtttctgg cccgggcggt     180

ccgggcaaaa tgatcaagcc tttctttcat tccctctctg aaaagtattc caacgtgata     240

ttccttgaag tagatgtgga tgactctcag gatgttgctt cagagtctga agtcaaaagc     300

atgccaacat tccagttttt taagaaggga caaaaggtgg gtgaattttc tggagccaat     360

aaggaaaagc ttgaagccac cattaatgaa ttcgtc                               396


<210>  31
<211>  396
<212>  DNA
<213>  Artificial

<220>
<223>  DNA sequence of a fusion protein between modified human
       thioredoxin (hTrx-delta-cys5) and the shortened and modified aptamer
       DD3-3.8-delta-cys together with introduced restiction sites

<400>  31
atggtgaagc agatcgagag caagactgct tttcaggaag ccttggacgc tgcaggtgat      60

aaacttgtag tagttgactt ctcagccacg tggggcggtc cgggatccgt tcggcattct     120

gcgcttcata tggcggtggg tccgttgtct tggccggcga gggtttctgg cccgggcggt 180

ccgggcaaaa tgatcaagcc tttctttcat tccctctctg aaaagtattc caacgtgata 240

ttccttgaag tagatgtgga tgactctcag gatgttgctt cagagtctga agtcaaaagc 300

atgccaacat tccagttttt taagaaggga caaaaggtgg gtgaattttc tggagccaat 360

aaggaaaagc ttgaagccac cattaatgaa ttcgtc 396

<210> 32
<211> 390
<212> DNA
<213> Artificial

<220>
<223> DNA sequence of a fusion protein between modified human
 thioredoxin (hTrx-delta-cys5) and the shortened aptamer DD3-1.10
 together with introduced restiction sites

<400> 32
atggtgaagc agatcgagag caagactgct tttcaggaag ccttggacgc tgcaggtgat 60

aaacttgtag tagttgactt ctcagccacg tggggcggtc cgggatcctc tcctattagt 120

attccgattg ggtttgttgt tcggcattgt gcgcttcata tggccccggg cggtccgggc 180

aaaatgatca agcctttctt tcattccctc tctgaaaagt attccaacgt gatattcctt 240

gaagtagatg tggatgactc tcaggatgtt gcttcagagt ctgaagtcaa aagcatgcca 300

acattccagt tttttaagaa gggacaaaag gtgggtgaat tttctggagc caataaggaa 360

aagcttgaag ccaccattaa tgaattcgtc 390

<210> 33
<211> 390
<212> DNA
<213> Artificial

<220>
<223> DNA sequence of a fusion protein between modified human
 thioredoxin (hTrx-delta-cys5) and the shortened and modified aptamer
 DD3-1.10-delta-cys together with introduced restiction sites

<400> 33
atggtgaagc agatcgagag caagactgct tttcaggaag ccttggacgc tgcaggtgat 60

aaacttgtag tagttgactt ctcagccacg tggggcggtc cgggatcctc tcctattagt 120

attccgattg ggtttgttgt tcggcattct gcgcttcata tggccccggg cggtccgggc 180

aaaatgatca agcctttctt tcattccctc tctgaaaagt attccaacgt gatattcctt 240

gaagtagatg tggatgactc tcaggatgtt gcttcagagt ctgaagtcaa aagcatgcca 300

acattccagt tttttaagaa gggacaaaag gtgggtgaat tttctggagc caataaggaa 360

aagcttgaag ccaccattaa tgaattcgtc 390

<210> 34
<211> 108
<212> DNA
<213> Artificial

<220>

<223>  Oligonucleotide for cloning the CCSL scaffold (description page
       22)

<400>  34
ggggaattcc atatgaagct ttacgcggct ctggagagcg aagtaagtgc gctcgaatcc      60

gaagtcgcga gtctcgaaag cgaaggcgcc ggtggaggcg gtccgggt                  108


<210>  35
<211>  96
<212>  DNA
<213>  Artificial

<220>
<223>  Second oligonucleotide for cloning the CCSL scaffold (description
       page 22)

<400>  35
cagcggaccg ggtggtgggc caaaagcaaa ctctctgcgg tcaaatccaa actcgcgagt      60

gtaaaatcca aactcgctgc gtacgaattc tctaga                                96


<210>  36
<211>  52
<212>  DNA
<213>  Artificial

<220>
<223>  Linker sequence (description page 22)

<400>  36
cggtccgggt ttggcccatg gttatgttaa cggggctcga ggtggcggtc cg              52


<210>  37
<211>  135
<212>  DNA
<213>  Artificial

<220>
<223>  Oligonucleotide for cloning the DD domains (description page 23)

<400>  37
aaagatctgc atatgtaggt aggttctaaa gttattctgt ttgaaggcgg tggcggccat      60

ggtggggctc gaggcggtcc gggtggttct aaagttattc tgtttgaagg tgaattcgtc     120

tagaaagggc ccaaa                                                      135


<210>  38
<211>  27
<212>  DNA
<213>  Artificial

<220>
<223>  Primer F1 (see description page 23)

<400>  38
ccagatctga tggtgaagca gatcgag                                          27


<210>  39
<211>  30
<212>  DNA
<213>  Artificial

<220>
<223>  Primer F2 (see description page 23)

<400>  39
ggcggtccgg gcaaaatgat caagcctttc                                        30


<210>  40
<211>  27
<212>  DNA
<213>  Artificial

<220>
<223>  Primer R1 (see description page 23)

<400>  40
gcccggaccg ccccacgtgg ctgagaa                                           27


<210>  41
<211>  24
<212>  DNA
<213>  Artificial

<220>
<223>  Primer R2 (see description page 23)

<400>  41
gaattcgact aattcattaa tggt                                              24


<210>  42
<211>  20
<212>  PRT
<213>  Artificial

<220>
<223>  DBD1 (see description page 23)

<400>  42

Pro Leu Thr Ala Val Phe Trp Leu Ile Tyr Val Leu Ala Lys Ala Leu
1               5                   10                  15


Val Thr Val Cys
            20


<210>  43
<211>  43
<212>  PRT
<213>  Artificial

<220>
<223>  All-7 (see description page 23)

<400>  43

Pro Leu Asn Phe Tyr Arg His Gly Phe Leu Pro Asn Ala Val Met Ala
1               5                   10                  15


Ser Met Leu Glu Val Gly Pro Trp Phe Glu Leu Leu Gly Pro Cys Gly
            20                  25                  30


Leu Ala Gly His Pro Leu Ser Ser Leu Arg Ile
        35                  40

<210>  44
<211>  19
<212>  PRT
<213>  Artificial

<220>
<223>  DD3-1.10 (see Fig. 7a)

<400>  44

Ser Pro Ile Ser Ile Pro Ile Gly Phe Val Val Arg His Cys Ala Leu
1               5                   10                  15

His Met Ala

<210>  45
<211>  30
<212>  DNA
<213>  Homo sapiens

<220>
<223>  hTrx-wt (see Fig. 3c)

<220>
<221>  CDS
<222>  (1)..(30)

<400>  45
gcc acg tgg tgt ggg cct tgc aaa atg atc                                30
Ala Thr Trp Cys Gly Pro Cys Lys Met Ile
1               5                   10

<210>  46
<211>  10
<212>  PRT
<213>  Homo sapiens

<220>
<223>  Coding Sequence of SEQ.ID.No. 45

<400>  46

Ala Thr Trp Cys Gly Pro Cys Lys Met Ile
1               5                   10

<210>  47
<211>  49
<212>  DNA
<213>  Artificial

<220>
<223>  hTrx-delta-cys5-L (see Fig. 3a)

<220>
<221>  CDS
<222>  (1)..(24)

<220>
<221>  CDS
<222>  (26)..(49)

<400>  47
gcc acg tgg ggc ggt ccg gga tcc c ccg ggc ggt ccg ggc aaa atg atc      49
Ala Thr Trp Gly Gly Pro Gly Ser   Pro Gly Gly Pro Gly Lys Met Ile
1               5                     10                  15

<210>  48
<211>  8
<212>  PRT
<213>  Artificial

<220>
<223>  Synthetic Construct

<400>  48

Ala Thr Trp Gly Gly Pro Gly Ser
1               5

<210>  49
<211>  8
<212>  PRT
<213>  Artificial

<220>
<223>  Synthetic Construct

<400>  49

Pro Gly Gly Pro Gly Lys Met Ile
1               5

<210>  50
<211>  35
<212>  PRT
<213>  Artificial

<220>
<223>  First part of SEQ.ID.No. 14

<400>  50

Met Ser Asp Lys Ile Ile His Leu Thr Asp Asp Ser Phe Asp Thr Asp
1               5                   10                  15

Val Leu Lys Ala Asp Gly Ala Ile Leu Val Asp Phe Trp Ala Glu Trp
            20                  25                  30

Ser Gly Pro
        35

<210>  51
<211>  81
<212>  PRT
<213>  Artificial

<220>
<223>  Second part of SEQ.ID.No. 14

<400>  51

Pro Gly Gly Pro Ser Lys Met Ile Ala Pro Ile Leu Asp Glu Ile Ala
1               5                   10                  15

Asp Glu Tyr Gln Gly Lys Leu Thr Val Ala Lys Leu Asn Ile Asp Gln
            20                  25                  30

Asn Pro Gly Thr Ala Pro Lys Tyr Gly Ile Arg Gly Ile Pro Thr Leu
        35                  40                  45

Leu Leu Phe Lys Asn Gly Glu Val Ala Thr Thr Lys Val Gly Ala Leu
    50                  55                  60

Ser Lys Gly Gln Leu Lys Glu Phe Leu Asp Ala Asn Leu Ala Glu Phe
65                  70                  75                  80

Gly

<210>  52
<211>  34
<212>  PRT
<213>  Artificial

<220>
<223>  First part of SEQ.ID.No. 15 and 16

<400>  52

Met Val Lys Gln Ile Glu Ser Lys Thr Ala Phe Gln Glu Ala Leu Asp
1               5                   10                  15

Ala Ala Gly Asp Lys Leu Val Val Val Asp Phe Ser Ala Thr Trp Gly
            20                  25                  30

Gly Pro

<210>  53
<211>  75
<212>  PRT
<213>  Artificial

<220>
<223>  Second part of SEQ.ID.No. 15

<400>  53

Pro Gly Gly Pro Gly Lys Met Ile Lys Pro Phe Phe His Ser Leu Ser
1               5                   10                  15

Glu Lys Tyr Ser Asn Val Ile Phe Leu Glu Val Asp Val Asp Asp Cys
            20                  25                  30

Gln Asp Val Ala Ser Glu Cys Glu Val Lys Cys Met Pro Thr Phe Gln
        35                  40                  45

Phe Phe Lys Lys Gly Gln Lys Val Gly Glu Phe Ser Gly Ala Asn Lys
    50                  55                  60

Glu Lys Leu Glu Ala Thr Ile Asn Glu Leu Val
65                  70                  75

<210>  54
<211>  75
<212>  PRT
<213>  Artificial

<220>
<223>  Second part of SEQ.ID.No. 16

<400>  54

Pro Gly Gly Pro Gly Lys Met Ile Lys Pro Phe Phe His Ser Leu Ser
1               5                   10                  15

Glu Lys Tyr Ser Asn Val Ile Phe Leu Glu Val Asp Val Asp Asp Ser
            20                  25                  30

Gln Asp Val Ala Ser Glu Ser Glu Val Lys Ser Met Pro Thr Phe Gln
        35                  40                  45

Phe Phe Lys Lys Gly Gln Lys Val Gly Glu Phe Ser Gly Ala Asn Lys
    50                  55                  60

Glu Lys Leu Glu Ala Thr Ile Asn Glu Leu Val
65                  70                  75

## Description

The present invention relates to scaffold proteins for recombinant peptide aptamers, especially to a modified variant of human thioredoxin, and to the aptamers themselves, especially apatamers binding to the transcription factor Stat3. The invention furthermore relates to Fusion Proteins of the scaffold protein and the aptamer, nucleic acids coding for these peptides or proteins, medicaments comprising same and their uses, especially in cancer therapy.

Crucial insights have been obtained into cellular signaling events, their aberrations in tumor cells and their contributions to cellular phenotypes. High expectations rest on the concept that these alterations can be exploited for the development of new therapeutic strategies, for example for the treatment of cancer. Genetic events leading to activation of oncogenes or inactivation of tumor-suppressor genes have been described to play an essential role in the transformation process. The proteins, which are structurally altered or affected in their extent of expression, are conceivably promising drug targets. Manipulation of the function or expression level of such proteins through drug interference might result in desirable therapeutic effects and minimal side effects. These aims are being pursued with many classes of drugs including small molecular weight compounds, antibodies, anti-sense molecules, therapeutic genes, peptides and proteins. The temporary inhibition of the function of an oncogene can result in tumor cell differentiation, an increase in chemosensitivity or the induction of apoptosis. Reversible tumorigenesis has been shown for various oncogenes with different functions in cellular signaling.

A subset of these molecular events can be targeted with conventional drugs and small molecular weight compounds have been developed, which act as enzyme inhibitors of e.g. kinases and proteases. However, other signaling related events, e.g. the transient assembly of high molecular weight multi-protein complexes, often evade the interference through small molecular weight molecules and are usually considered as non-drugable. Peptides have been taken into consideration as tools to affect such functions. Peptides have important advantages when they are envisaged as drugs. No prior knowledge of the structure of the target protein is required and their binding ability is not limited to preexisting small molecular weight compound binding pockets. Peptides are potentially able to selectively block one specific function of their target protein, e.g. a substrate-binding site, or the interaction site of an other binding partner. This option complements knock-out techniques that target the activity or expression of the entire gene product. These properties make peptides interesting candidates for the development of highly target specific and less toxic cancer drugs.

Peptide aptamers are short peptides, usually 12 to 20 amino acids in length that can be selected from a random peptide library. They specifically bind to a given target protein under intracellular conditions. Each oligopeptide of the library is displayed in a unique conformation and the library is large enough to contain particular peptides able to recognize and bind a large variety of target structures. Selection occurs through screening of a high complexity peptide library. This can be done intracellularly using yeast or mammalian cells. Alternatively, screening can be performed extracellularly using the phage display technology. The interference of peptide aptamers with the function of crucial viral or cellular regulatory proteins has been shown and validated the concept that aptamers can be employed as inhibitors of crucial biological processes. To optimize the binding properties to their target structures, peptide aptamers are usually presented in constrained configurations. They are embedded in scaffold constructs and have been defined as "combinatorial protein molecules consisting of a variable peptidic sequence within a constant scaffold protein" (Colas et al., Nature 1996;380(6574):548-50). Peptide aptamers inserted into scaffolds are more stable than free peptides and can be delivered more easily into cells.

The green fluorescent protein, glutathione-S-transferase and staphylococcal nuclease have been used as carriers of peptide aptamers and a derivative of the intracellular protease inhibitor stefin A has been described as a conformational stabilizer for peptides. In addition, isolated protein domains, e.g. the Z domain of the staphyloccocal protein A ("affibodies"), lipocalin ("anticalins") or ankryin repeats have been taken into consideration. However, most frequently peptide aptamers have been inserted into the bacterial thioredoxin (bTrx) protein. Bacterial thioredoxin can be easily expressed in *E. coli* or in yeast cells, it is highly stable and has a relatively small size of 109 amino acids. Insertion of the peptide aptamers into the active site of bTrx results in their exposure on the surface of the scaffold and the inactivation of the enzymatic function of bTrx. Despite these advantageous properties of bTrx, most studies have used intracellular expression of peptide aptamers by the transfection of DNA constructs to analyze the effects of the selected aptamers on cellular processes. Nevertheless, if peptide aptamers are developed further for therapeutic purposes, it will become necessary to deliver them as recombinant proteins. Thus it was the purpose of this invention to identify a suitable solution allowing aptamers to be delivered as recombinant proteins. Essential for these therapeutic *in vivo* applications, however, are high recombinant expression levels, robust purification properties and preferably also low side effects (e.g. immunogenicity).

Even though bacterial thioredoxin is in principle a starting point for this kind of application known in the art, there are problems connected with bTrx, which limit the therapeutic usefulness of peptide aptamers inserted into bTrx. On one hand there were difficulties in binding and on the other hand the bTrx scaffold was difficult to purify in a monomeric form when aptamers were inserted, as aggregation of these recombinant proteins into high molecular weight complexes occur during the purification process. In addition, the protein is structurally highly related to its human orthologue, but exhibits only 30% sequence homology. For this reason, it is most likely immunogenic in humans. These observations indicate the need for further improvements before the *in vivo* application of peptide aptamers can be seriously approached.

This problem was solved by a scaffold protein suitable for the use in combination with a recombinant peptide aptamer in which at least one cysteine of the wild type is replaced by a non-sulphur-containing amino acid. Preferably, the scaffold protein according to the invention is thioredoxin, preferably is bacterial thioredoxin or human thioredoxin, whereby in apreferred embodiment of the invention the non-sulphur-containing amino acid or acids is/are selected from glycine, serine, valine, leucine, isoleucin, alanine, lysine, or threonine, preferably is/are selected from glycine or serine.

The exchange of one or more cysteins in the scaffold protein, especially in the bacterial and human thioredoxin, resulted in a surprisingly high effects. On one hand the purification was strongly enhanced with a high amount of monomers being isolated opposed to the oligomers of high molecular weight seen without the modification. On the other hand in contrast to the wild-type, which, when bound to a peptide aptamer, did aggregate and did not allow proper cellular uptake, the modified scaffold protein, especially the modified bacterial or human thioredoxin, allowed for a n effective uptake into the cells evevn when loaded/bound to a peptide aptamer. In addition high recombinant expression levels are achieved when the Scaffold Protein is expressed as Fusion Protein together with a peptide aptamer.

According to the invention, the aforementioned proteins or peptides of the invention and those being described hereafter can be modified, i.e. in the form of derivatives of those proteins. Derivatives of proteins are fragments and variants thereof. Preferably, derivatives of said proteins or peptides are functional derivatives. A "fragment" in the context of a protein is to be understood as a truncated protein, i.e. a protein which is N-terminally, C-terminally or intrasequentially truncated compared to the amino acid sequence of the original (wild-type) protein. Especially, fragments including an antigenic epitope are preferred. A "variant" in the context of a protein refers to a protein having an amino acid sequence which differs from the original sequence in one or more mutation(s), such as one or more substituted, inserted and/or deleted amino acid(s), and having a sequence homology of at least 60%, preferably at least 70%, more preferably at least 80%, equally more preferably at least 85%, even more preferably at least 90% and most preferably at least 97%, to the wild-type sequence.

In a very preferred embodiment of the Scaffold Protein according to the invention the scaffold protein comprises a protein sequence selected from general sequence I or general sequence II
General sequence I: 
M S D K I I H L T D D S F D T D
V L K A D G A I L V D F W A E W
 
X1 G P X2 K M I A P I L D E I A D
E Y Q G K L T V A K L N I D Q N
P G T A P K Y G I R G I P T L L
L F K N G E V A T T K V G A L S
K G Q L K E F L D A N L A E F G
 or
General Sequence II 
M V K Q I E S K T A F Q E A L D
A A G D K L V V V D F S A T W X1
G P X2 K M I K P F F H S L S E K
Y S N V I F L E V D V D D X3 Q D
V A S E X4 E V K X5 M P T F Q F F
K K G Q K V G E F S G A N K E K
L E A T I N E L V
wherein at least one of X1, or X2, or at least one of X1, X2, X3, X4 or X5 is selected from glycine, serine, valine, leucine, isoleucin, alanine, lysine, or threonine, while the remaining are cystein. This Scaffold Protein may also be present as a variant of the the abovedescribed Scaffold Protein of general formulas I or II.

In another highly preferred embodiment of the Scaffold Protein according to the invention X1 and X2 are selected from serine or glycine, while X3, X4 and X5 are selected from cysteine, glycine, serine, valine, leucine, isoleucin, alanine, lysine, or threonine, preferably are selected from cysteine, serine or glycine, most preferably are selected from serine or glycine.

In another highly preferred embodiment of the Scaffold Protein according to the invention the scaffold protein comprises an amino acid sequence identical to or showing a similarity of 85% to the SEQ.ID.Nos. 2, 4 or 5; preferably comprises an amino acid sequence identical to or showing a similarity of 90% to the SEQ.ID.Nos. 2, 4 or 5; more preferably comprises an amino acid sequence identical to or showing a similarity of 95% to the SEQ.ID.Nos. 2, 4 or 5.

Another aspect of the invention relates to peptide aptamers, in which at least one cysteine of the wild type of this peptide aptamer is replaced by a non-sulphur-containing amino acid. Preferably, in one embodiment of the peptide aptamer according to invention the non-sulphur-containing amino acid is selected from glycine, serine, valine, leucine, isoleucin, alanine, lysine, or threonine, preferably is selected from glycine or serine.

This part of the invention was investigated in the context of the search for a solution to the overall underlying purpose of this invention to allow aptamers to be delivered as recombinant proteins in *in vivo* applications, especially in regards to robust purification properties. There it was surprisingly found that also in regards to peptide aptamers it turned out to be very advantageous to replace at least one cysteine-if applicable-by a non-sulphur-containing amino acid, preferably serine or glycine, again enhancing the isolation of monomers opposed to oligomerization found more frequently with non-modified peptide aptamers.

In a preferred embodiment the invention refers to a peptide aptamer binding to the transcription factor Stat3 according to the invention, comprising an amino acid sequence according to SEQ.ID.No. 6.

In another preferred embodiment of the peptide aptamer binding to the transcription factor Stat3 according to the invention, the peptide aptamer is comprising an amino acid sequence of 15 to 30 amino acids being comprised within the amino acid sequence SEQ.ID.Nos. 6 or 7.

In another preferred embodiment the invention refers to a peptide aptamer binding to the transcription factor Stat3 according to the invention, comprising an amino acid sequence identical to or showing a similarity of 85% to the SEQ.ID.Nos. 8, 9, 10, 11, 12 or 13; preferably comprising an amino acid sequence identical to or showing a similarity of 90% to the SEQ.ID.Nos. 8, 9, 10, 11, 12 or 13; more preferably comprises an amino acid sequence identical to or showing a similarity of 95% to the SEQ.ID.Nos. 8, 9, 10, 11, 12 or 13.

In another preferred embodiment the invention refers to a peptide aptamer binding to the transcription factor Stat3 according to the invention consisting of the amino acid sequence of SEQ.ID.Nos. 8, 9, 10, 11, 12 or 13; preferably consisting of the amino acid sequence of SEQ.ID.Nos. 8, 9, 10 or 11; more preferably consisting of the amino acid sequence of SEQ.ID.Nos. 10 or 11.

In another aspect the invention refers to a Fusion Protein comprising a Scaffold Protein according to the invention and a protein or peptide bound to the Scaffold Protein either at the 3' or 5' end of the protein sequence of the Scaffold Protein or by being inserted into the protein sequence of the Scaffold Protein. Preferably, in an embodiment the of the Fusion Protein according to the invention the protein or peptide is bound to the Scaffold Protein by being inserted into the protein sequence of the Scaffold Protein, and the protein is expressing a dominant negative phenotype in a cell. Preferably, the binding between Scaffold Protein and peptide aptamer is achieved through peptide bonds.

In a preferred embodiment of the Fusion Protein according to the invention the protein or peptide being bound to the Scaffold Protein is a peptide aptamer.

In another preferred embodiment of the Fusion Protein according to the invention the peptide aptamer is bound to the Scaffold Protein by being inserted into the protein sequence of the Scaffold Protein.

In another preferred embodiment of the Fusion Protein according to the invention the peptide aptamer is a peptide comprising 5 to 35 amino acids, preferably 10 to 30 amino acids, most preferably 12 to 25 amino acids, most preferably consisting of 12 to 25 amino acids.

In another preferred embodiment of the Fusion Protein according to the invention the Fusion Protein comprises a protein sequence selected from general sequence III or general sequence IV or general sequence V
General sequence III: 
M S D K I I H L T D D S F D T D
V L K A D G A I L V D F W A E W
X1 Y1..Y2 X2 K M I A P I L D E I A D
 
E Y Q G K L T V A K L N I D Q N
P G T A P K Y G I R G I P T L L
L F K N G E V A T T K V G A L S
K G Q L K E F L D A N L A E F G
 or
General Sequence IV: 
M V K Q I E S K T A F Q E A L D
A A G D K L V V V D F S A T W X1
Y1..Y2 X2 K M I K P F F H S L S E K
Y S N V I F L E V D V D D X3 Q D
V A S E X4 E V K X5 M P T F Q F F
K K G Q K V G E F S G A N K E K
L E A T I N E L V
 or
General Sequence V: 
M V K Q I E S K T A F Q E A L D
A A G D K L V V V D F S A T W X1
Y1..Y2 P G X2 K M I K P F F H S L S
E K Y S N V I F L E V D V D D X3
Q D V A S E X4 E V K X5 M P T F Q
F F K K G Q K V G E F S G A N K
E K L E A T I N E L V
wherein
at least one of X1, or X2, or X1, X2, X3, X4 or X5 is selected from glycine, serine, valine, leucine, isoleucin, alanine, lysine, or threonine, while the remaining are cystein; and
wherein Y1..Y2 signifies an amino acid sequence comprising the amino acid sequence of the peptide aptamer and optionally the amino acid sequence Gly-Pro bound to one or both ends of the peptide aptamer and/or optionally additional amino acids.

Here it is highly preferred if X1 and X2 are selected from serine or glycine, while X3, X4 and X5 are selected from cysteine, glycine, serine, valine, leucine, isoleucin, alanine, lysine, or threonine, preferably are selected from cysteine, serine or glycine, most preferably are selected from serine or glycine. It is also highly preferred if Y1..Y2 signifies an amino acid sequence comprising an amino acid sequence identical to or showing a similarity of 85% to the SEQ.ID.Nos. 8, 9, 10, 11, 12 or 13; preferably comprising an amino acid sequence identical to or showing a similarity of 90% to the SEQ.IDs.No. 8, 9, 10, 11, 12 or 13; more preferably comprises an amino acid sequence identical to or showing a similarity of 95% to the SEQ.ID.Nos. 8, 9, 10, 11, 12 or 13; highly preferably consists of an amino acid sequence identical to or showing a similarity of 95% to the SEQ.ID.Nos. 8, 9, 10 or 11; most preferably consists of an amino acid sequence identical to the SEQ.ID.Nos. 10 or 11.

In another preferred embodiment of the Fusion Protein according to the invention the Fusion Protein comprises an amino acid sequence identical to or showing a similarity of 85% to the SEQ.ID.Nos. 17, 18, 19, 20, 21 or 22; preferably comprising an amino acid sequence identical to or showing a similarity of 90% to the SEQ.IDs.No. 17, 18, 19, 20, 21 or 22; more preferably comprises an amino acid sequence identical to or showing a similarity of 95% to the SEQ.ID.Nos. 17, 18, 19, 20, 21 or 22; highly preferably consists of an amino acid sequence identical to or showing a similarity of 95% to the SEQ.ID.Nos. 17, 18, 19, 20, 21 or 22; most preferably consists of an amino acid sequence identical to the SEQ.ID.Nos. 17, 18, 19 or 20 or 19 or 20.

Another aspect of the invention refers to a nucleic acid coding for a Scaffold Protein according to the invention. In a preferred embodiment of the nucleic acid according to the invention, the nucleic acid is coding for a protein according to SEQ.ID.Nos. 2, 4 or 5, preferably to SEQ.ID. Nos. 4 or 5, most preferably to SEQ.ID.No. 5.

In another preferred embodiment of the nucleic acid according to the invention the nucleic acid comprises a nucleic acid sequence identical to or showing a similarity of 85% to SEQ.ID.Nos. 23 or 24; preferably is comprising a nucleic acid sequence identical to or showing a similarity of 90% to SEQ.ID.Nos. 23 or 24; more preferably comprises a nucleic acid sequence identical to or showing a similarity of 95% to the SEQ.ID.Nos. 23 or 24; highly preferably consists of a nucleic acid sequence identical to or showing a similarity of 95% to the SEQ.ID.Nos. 23 or 24; most preferably consists of a nucleic acid sequence identical to the SEQ.ID.No. 24.

Another aspect of the invention refers to a nucleic acid coding for a peptide aptamer according to the invention. In a preferred embodiment of the nucleic acid according to the invention, the nucleic acid is coding for a protein/peptide according to SEQ.ID.Nos. 6, 8, 9, 10, 11, 12 or 13, preferably to SEQ.ID. Nos. 8, 9, 10 or 11, most preferably to SEQ.ID.Nos. 10 or 11.

In another preferred embodiment of the nucleic acid according to the invention, the nucleic acid comprises a nucleic acid sequence identical to or showing a similarity of 85% to SEQ.ID.Nos. 25, 26, 27, 28 or 29; preferably is comprising a nucleic acid sequence identical to or showing a similarity of 90% to SEQ.ID.Nos. 25, 26, 27, 28 or 29; more preferably comprises a nucleic acid sequence identical to or showing a similarity of 95% to the SEQ.ID.Nos. 25, 26, 27, 28 or 29; highly preferably consists of a nucleic acid sequence identical to or showing a similarity of 95% to the SEQ.ID.Nos. 25, 26, 27, 28 or 29; most preferably consists of a nucleic acid sequence identical to the SEQ.ID.Nos. 28 or 29.

Another aspect of the invention refers to a nucleic acid coding for a Fusion Protein according to the invention. In a preferred embodiment of the nucleic acid according to the invention, the nucleic acid is coding for a Fusion Protein according to SEQ.ID.Nos. 17, 18, 19, 20, 21 or 22, preferably to SEQ.ID. Nos. 17, 18, 19 or 20, most preferably to SEQ.ID.Nos. 19 or 20.

In another preferred embodiment of the nucleic acid according to the invention, the nucleic acid comprises a nucleic acid sequence identical to or showing a similarity of 85% to SEQ.ID.Nos. 30, 31, 32 or 33; preferably is comprising a nucleic acid sequence identical to or showing a similarity of 90% to SEQ.ID.Nos. 30, 31, 32 or 33; more preferably comprises a nucleic acid sequence identical to or showing a similarity of 95% to the SEQ.ID.Nos. 30, 31, 32 or 33; highly preferably consists of an amino acid sequence identical to or showing a similarity of 95% to the SEQ.ID.Nos. 30, 31, 32 or 33; most preferably consists of an amino acid sequence identical to the SEQ.ID.Nos. 30 or 31.

The subjects according to this invention, the Scaffold Proteins, the peptide aptamers, the Fusion Proteins and the nucleic acids are -as far as predictable - physiologically harmless. Accordingly another aspect of this invention is a medicament comprising a Scaffold Protein according to the invention, a peptide aptamer according to the invention, a Fusion Protein according to the invention and/or a nucleic acid according to the invention; and optionally at least one pharmaceutically acceptable carrier.

The inventive medicament, preferably a pharmaceutical composition, typically comprises a safe and effective amount of the compounds according to the invention (the Scaffold Proteins, the peptide aptamers, the Fusion Proteins and the nucleic acids) as defined above. As used here, "safe and effective amount" means an amount of the compounds as defined above, that is sufficient to significantly induce a positive modification of a condition to be treated, for example of a tumor, autoimmune diseases, etc. At the same time, however, a "safe and effective amount" is small enough to avoid serious side-effects, that is to say to permit a sensible relationship between advantage and risk. The determination of these limits typically lies within the scope of sensible medical judgment. A "safe and effective amount" of the compounds according to the invention as defined above will vary in connection with the particular condition to be treated and also with the age and physical condition of the patient to be treated, the severity of the condition, the duration of the treatment, the nature of the accompanying therapy, of the particular pharmaceutically acceptable carrier used, and similar factors, within the knowledge and experience of the accompanying doctor. The medicament according to the invention can be used according to the invention for human and also for veterinary medical purposes, as a pharmaceutical composition.

The medicament according to the invention typically contains a pharmaceutically acceptable carrier. The expression "pharmaceutically acceptable carrier" as used herein preferably includes the liquid or non-liquid basis of the inventive medicament. If the inventive medicament is provided in liquid form, the carrier will typically be pyrogen-free water; isotonic saline or buffered (aqueous) solutions, e.g phosphate, citrate etc. buffered solutions. Particularly for injection of the inventive medicament, a buffer, preferably an aqueous buffer, may be used, containing a sodium salt. The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects. Reference media are e.g. in *"in vivo"* methods occurring liquids such as blood, lymph, cytosolic liquids, or other body liquids, or e.g. liquids, which may be used as reference media in *"in vitro"* methods, such as common buffers or liquids. Such common buffers or liquids are known to a skilled person. Ringer-Lactate solution is particularly preferred as a liquid basis.

However, one or more compatible solid or liquid fillers or diluents or encapsulating compounds may be used as well, which are suitable for administration to a person. The term "compatible" as used here means that the constituents of the inventive medicament are capable of being mixed with the compound according to the invention as defined above in such a manner that no interaction occurs which would substantially reduce the pharmaceutical effectiveness of the inventive medicament under usual use conditions. Pharmaceutically acceptable carriers must, of course, have sufficiently high purity and sufficiently low toxicity to make them suitable for administration to a person to be treated. Some examples of compounds which can be used as pharmaceutically acceptable carriers or constituents thereof are sugars, such as, for example, lactose, glucose and sucrose; starches, such as, for example, corn starch or potato starch; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulfate; vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid.

The choice of a pharmaceutically acceptable carrier is determined in principle by the manner in which the inventive medicaments are administered. The inventive medicaments can be administered, for example, systemically. Routes for administration include, for example, transdermal, oral, parenteral, including subcutaneous or intravenous injections, topical and/or intranasal routes. The suitable amount of the inventive medicament to be administered can be determined by routine experiments with animal models. Such models include, without implying any limitation, rabbit, sheep, mouse, rat, dog and non-human primate models. Preferred unit dose forms for injection include sterile solutions of water, physiological saline or mixtures thereof. The pH of such solutions should be adjusted to about 7.4. Suitable carriers for injection include hydrogels, devices for controlled or delayed release, polylactic acid and collagen matrices. Suitable pharmaceutically acceptable carriers for topical application include those which are suitable for use in lotions, creams, gels and the like. If the inventive medicament is to be administered perorally, tablets, capsules and the like are the preferred unit dose form. The pharmaceutically acceptable carriers for the preparation of unit dose forms which can be used for oral administration are well known in the prior art. The choice thereof will depend on secondary considerations such as taste, costs and storability, which are not critical for the purposes of the present invention, and can be made without difficulty by a person skilled in the art.

Also included in the present invention are methods of treating a disease connected to the transcription factor Stat3 by administering to a patient in need thereof a pharmaceutically effective amount of an inventive medicament, or a pharmaceutically effective amount of an inventive compound (Scaffold Proteins, peptide aptamer, Fusion Proteins or nucleic acid). Such a method typically comprises an optional first step of preparing the inventive medicament, or the inventive compound, and a second step, comprising administering a pharmaceutically effective amount of said inventive medicament, or said inventive compound.

Stat3 is a transcription factor closely related to tumor cells. In glioma cells constitutively activated Stat3 promotes cell-cycle progression and survival, stimulates angiogenesis and impairs immunological responses to cancer cells. Stat3 activation is accompanied by an increased expression of cell-cycle and survival regulators, such as cyclin D1, c-myc, Bcl-XL and survivin. Therefore, Stat3 is a preferred target for therapeutic intervention. It does not seem to be essential for the survival of many normal cells, but it is indispensable for many different tumor types. Efficient inhibition of Stat3 provides an attractive option, especially for targeted cancer therapy.

Another aspect of the invention refers to the use of a peptide aptamer according to the invention, of a Fusion Protein according to the invention, and/or of a nucleic acid according to the invention for the preparation of a medicament for the treatment or prevention of a disease, of symptoms of a disease or causes for a disease connected to the transcription factor Stat3. It is prefered in this use according to the invention if the disease is cancer, an autoimmuno-disease, chronic inflammation, psoriasis, a liver disease, preferably is cancer.

In another preferred embodiment of the use according to the invention the treatment is suppression of tumor cells, suppression of the metastasing potential of tumor cells, activation of immuno cells interacting with tumor cells, removing the block of differentiation of dendritic celss, or inhibition of Stat3 functions.

Prevention or treatment of the disease and induction or enhancement of the immune response may be carried out by using different inventive medicaments as defined above in a time staggered manner.

According to another embodiment, the present invention also provides kitscomprising a composition comprising a Scaffold Protein according to the invention, a peptide aptamer according to invention, a Fusion Protein according to the invention, a nucleic acid according to the invention, and/or a medicament according to the invention and optionally technical instructions with information on the administration and dosage of the composition and/or the medicament. The technical instructions may contain information about administration and dosage of the inventive compounds, and/or the inventive medicament. Such kits may applied e.g. for any of the above mentioned applications or uses. The kits may also be applied for the use of of a peptide aptamer according to the invention, of a Fusion Protein according to the invention, and/or of a nucleic acid according to the invention for the preparation of a medicament for the treatment or prevention of a disease, of symptoms of a disease or causes for a disease connected to the transcription factor Stat3

Another aspect of the invention refers to a method for improving the efficacy and/or purification parameters of a peptide aptamer and/or of a Fusion Protein between a peptide aptamer and a Scaffold Protein, wherein in the Scaffold protein and/or in the peptide aptamer at least one cysteine of the wild type is replaced by a non-sulphur-containing amino acid, selected from glycine, serine, valine, leucine, isoleucin, alanine, lysine, or threonine, preferably selected from glycine or serine.

### Figures

The following Figures are intended to illustrate the invention further. They are not intended to limit the subject matter of the invention thereto.
**Figure 1****:**

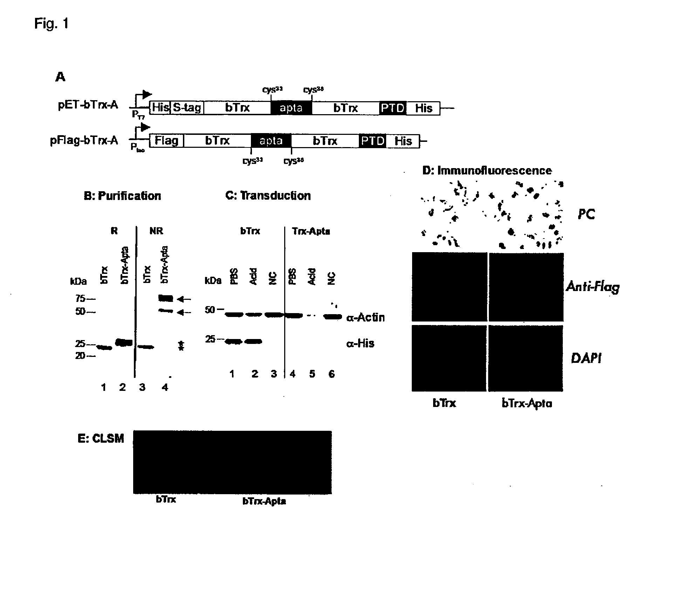

   **Figure 1A****)** depicts a schematic presentation of bacterial expression vectors encoding the bTrx scaffold. The position of the aptamer between the two cysteine residues is indicated. The fusion proteins are encoded by two different vectors pET30 (Novagen) with a T7 promoter and pFlag (Sigma, Taufkirchen, Germany) with a tac promoter for IPTG inducible expression of the recombinant proteins. A PTD of nine arginine residues and one or two His tags for purification on nickel columns have been added. The Flag-tag served for specific antibody recognition in immuno florescence experiments.
   **Figure 1B****)** depicts stained gels after protein puification. Bacterial thioredoxin without (bTrx) and with an aptamer insert (bTrx-Apta) were expressed in bacteria and lysates were obtained. The recombinant proteins were purified by nickel column chromatography and 2 µg were separated by gel electrophoresis under reducing (R) and non-reducing (NR) conditions. The proteins were visualized by staining with Coomassie blue. An asterisk indicates the position of the bTrx or bTrx-Apta monomer. Arrows indicate the formation of dimers and other multimers under non-reducing conditions.
   **Figure 1C)** depicts a gel showing the transduction of purified bTrx and bTrx-Apta proteins into NIH3T3 cells. The purified proteins were added to the medium of the cells. After 4 h the cells were washed with PBS, or with PBS and acid (0.2 M acetic acid in PBS) to remove aptamers associated with the outer membrane of the cells. Whole cell extracts were prepared, the proteins (20 µg) were separated by gel electrophoresis and the recombinant proteins were detected with an antibody specific for the His-tag. The negative control represents lysates of non-transduced cells. The detection of actin served as a loading control.
   **Figure 1D)** depicts the detection of transduced proteins by immunofluorescence analysis. After transduction of NIH3T3 cells with bTrx and bTrx-Apta for 4 h, the cells were fixed and stained with an anti-Flag antibody to detect the recombinant proteins. Nuclei were stained with DAPI. PC; phase contrast imaging.
   **Figure 1E)** depicts confocal laser scanning images of cells transduced for 4 h with bTrx (left) or with peptide aptamer bTrx-AII-7 (middle and right panel). After fixation of the cells a Flag antibody was used for detection of the proteins using a standard immunofluorescence protocol.
**Figure 2****:**

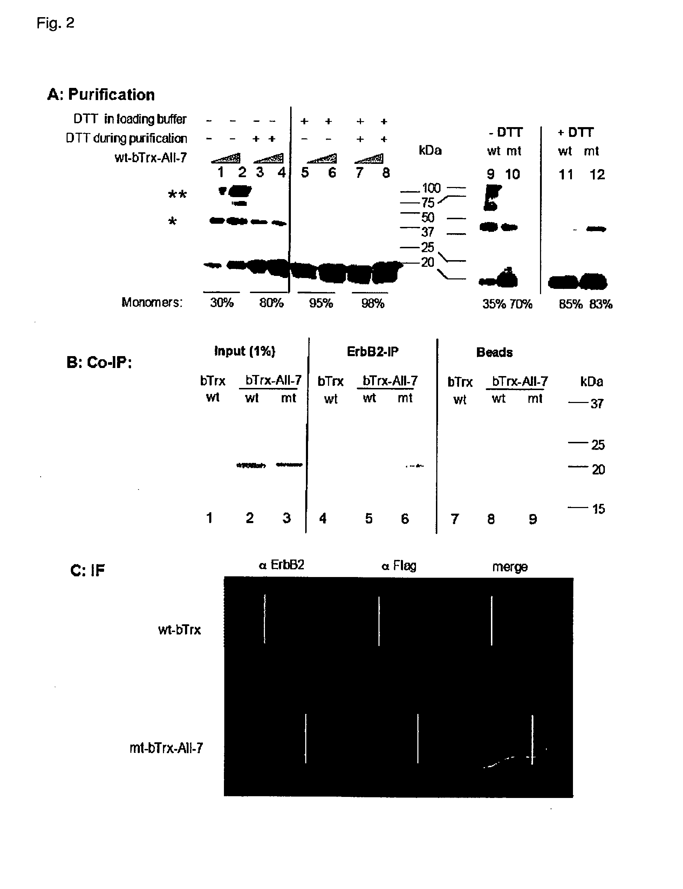

   **Figure 2A****)** depicts the effect of DTT on the purification and the effect of the elimination of cysteine residues from the bTrx scaffold protein. The aptamer All-7 inserted into the wild-type bTrx scaffold (wt bTrx-AII-7) was expressed in *E. coli,* purified using the His tag on a FPLC system, dialyzed and 1 and 2 µg were analyzed by gel electrophoresis (lane 1-2). DTT (0.3 M) was added to the loading buffer (lane 5-6) or during the first dialysis step of the purification procedure (lane 3-4) or both (lane 7-8) to disrupt disulfide bonds. The presence of dimers and multimers, is indicated by asterisks. The bTrx scaffold was mutated by exchanging Cys33 and Cys36 to serines (mt). Purification of All-7 in this mutated scaffold (lane 10, 12) was compared to wild-type bTrx (lane 9, 11) and analyzed in the presence or absence of 0.3 M DTT in the loading buffer (right panel). The fraction of monomers detected in each lane was densitometrically determined, normalized to the total amount of protein present in each lane and indicated below the blots.
   **Figure 2B****):** The binding of wt bTrx-AII-7 and mt bTrx-AII-7 to the ErbB2 receptor was compared in co-IP experiments. ErbB2 enriched cell lysates were mixed with equal amounts of the bTrx aptamer proteins and the complexes were immunoprecipitated using an ErbB2 antibody and magnetic beads. Complex formation of the aptamer with the receptor was analyzed by Western blotting with a Flag specific antibody (middle). Input fractions (left) indicate the amount of peptide aptamer added in each IP reaction. Bead fractions (right) show the amount of peptide aptamers unspecifically binding to the beads.
   **Figure 2C****):** Transduction of the wild-type bTrx scaffold or the mutated scaffold with aptamer All-7 into cells. The ErbB2 (red) and the transduced proteins (green) were detected with fluorescent antibodies. Co-localization of the aptamer and the receptor is indicated by yellow (light) fluorescence in the overlay.
**Figure 3****:**

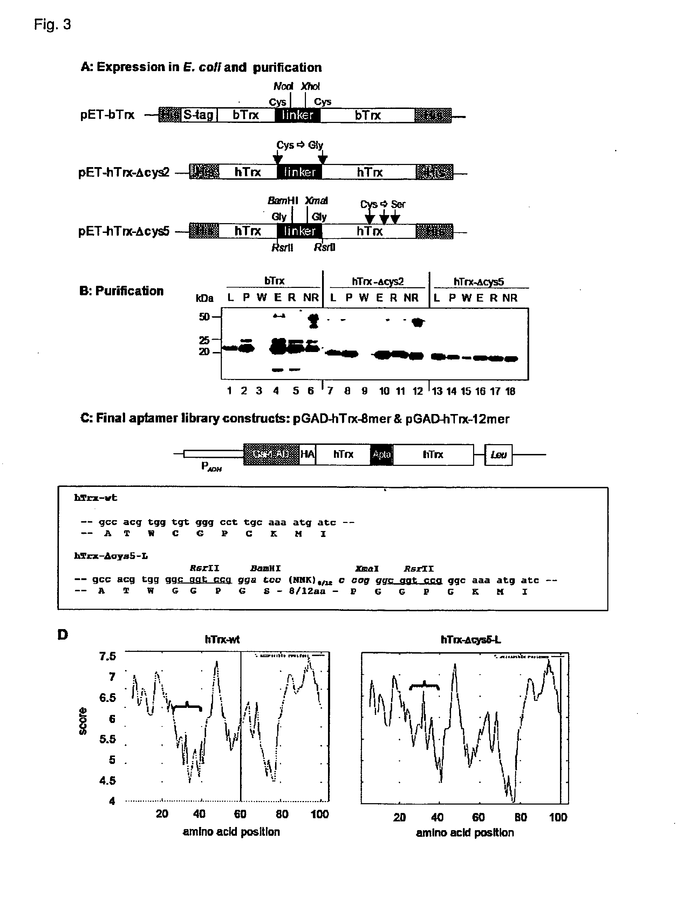

   **Figure 3A****)** shows the optimization of the hTrx scaffold. It depicts maps showing the original bTrx based fusion construct (pET-bTrx), the hTrxbased construct in which two cysteines in the active site, Cys32 and Cys35, have been replaced by glycines (pET-hTrx-_cys2) and the hTrx scaffold with three additional cysteine -> serine substitutions (pET-hTrx-_cys5).
   **Figure 3B****)** shows a gel with purification properties of bTrx (lanes 1 to 6), hTrx-_cys2 (lanes 7 to 12) and hTrx-_cys5 (lanes 13 to 18) scaffold protein. Shown are whole bacterial cell lysates under native conditions (L) and lysates prepared from the cell pellet (P) under denaturing conditions. Pellets were solubilized and loaded onto nickelsepharose columns. After washing (W, wash fraction) proteins were eluted (E, elution fraction) from the column and finally dialysed. After dialysis, the proteins were analyzed by gel electrophoresis in the presence (reducing conditions, R) or absence (non-reducing conditions, NR) of -mercaptoethanol.
   **Figure 3C)** shows the vector used for the cloning of a novel 8mer or 12mer peptide aptamer library. This vector is based on pGAD-T7 (Clontech). The sequences of the active site of the original hTrx as well as of the novel hTrx-scaffold with cloning sites *Bam*H I and *Xma* I for directional insertion of the randomized peptide aptamer library are shown below.
   **Figure 3D****)** shows an accessibility analysis using the ProtScale program (30) of the peptide aptamer insertion site (black brace) in the wt hTrx molecule or in the modified hTrx with a linker for cloning of randomized sequences. The y-axis indicates the probability that the amino acids indicated on the x-axis are exposed to the outside of the molecule. Restriction sites were chosen that showed the highest probability for the formation of an accessible protrusion in the scaffold.
**Figure 4****:**
   The aptamers DD3, DBD1 binding to Stat3 and aptamer All-7 binding the ErbB2 receptor were originally isolated from the 20mer bTrx-aptamer library. The aptamers were cloned into the pGAD-hTrx vector (encoding leucine) shown in Fig. 3C. The prey construct and the corresponding bait construct (Stat3 or ErbB2 fused to the Gal4-DBD, encoding tryptophan) were transformed into yeast. Binding of aptamers to their targets was verified in a yeast-two-hybrid assay by plating serial dilutions (100, 10-1, 10-2, 10-3) on selective media lacking leucine (L), tryptophan (T), histidine (H) and/or adenin (A). If weak interactions occur the cells are able to grow on -LTH, stronger interactions allow growth on -LTA. The binding efficiency was also determined using -galactosidase assays as shown on the right (indicated in Miller Units). To obtain mean values, at least 5 different colonies were measured at least 3 times. The interaction between p53 or LaminC with the SV40-LargeT antigen served as a positive and negative control, respectively.
**Figure 5****:**

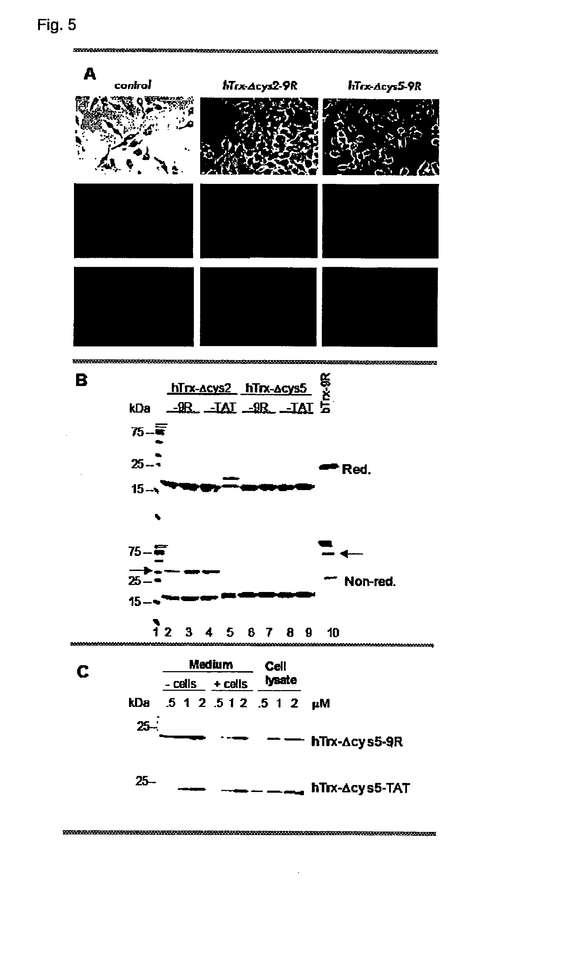

   **Figure 5A****)** depicts Protein transduction properties of recombinant hTrx scaffold proteins. Immuno fluorescence analysis of transduced hTrx-_cys2-9R and hTrx-_cys5-9R proteins. Cells were transduced for 4h with 1 µM of the indicated scaffold proteins, fixed and stained with a Flag antibody (green). Non-transduced cells served as a negative control (right). Nuclei were stained with DAPI. To compare the fluorescence intensities as a measure for the amount of transduced proteins, all pictures were taken with the same camera settings.
   **Figure 5B****)** depicts a gel. The hTrx scaffold was fused to a protein transduction domain comprised of 9 arginines (hTrx-9R) or to the PTD sequence derived from the HIV-Tat protein (hTrx-TAT). The proteins were expressed in bacteria and for each sample two batches were purified and analyzed by gel electrophoresis (1 µg per lane) under reducing (upper gel) and non-reducing (lower gel) conditions. For comparison, the purified bTrx-9R scaffold was included on the gel. Dimers are indicated by arrows.
   **Figure 5C****)** depicts a gel. Increasing amounts (0.5-2 µM) of purified hTrxDcys5-9R and hTrxDcys5-TAT were incubated in the medium without cells (control) or with NIH3T3 cells. The remaining proteins in the medium were analyzed after 4 h. Also cell lysates were prepared to confirm uptake of the proteins into the cells.
**Figure 6****:**

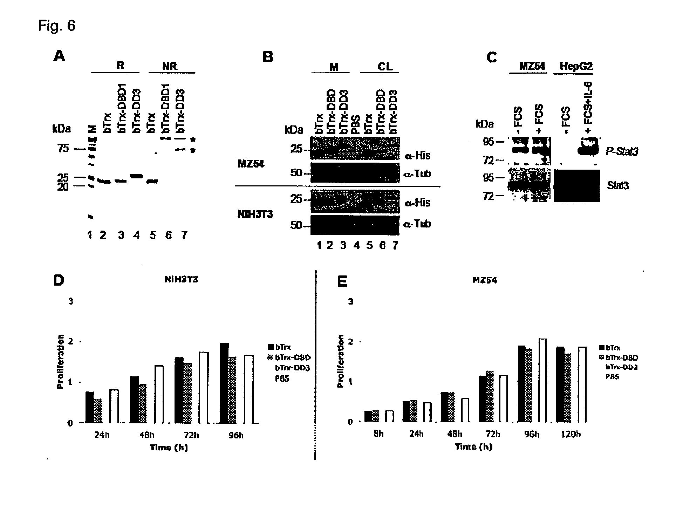

   **Figure 6A****)** depicts the transduction characteristics of aptamers inserted into the bTrxScaffold. The bTrx scaffold and the scaffold with aptamers DBD1 (bTRx-DBD1) and DD3 (bTrx-DD3) were purified an analyzed under reducing (R) and non-reducing (NR) conditions by Coomassie staining. 1 µg of purified protein was loaded in all lanes. Dimers and multimers are indicated with asterisks.
   **Figure 6 B)** depicts the transduction of 1 µM bTrx, bTrx-DBD1 and bTrx-DD3 into NIH3T3 cells and MZ54 glioblastoma cells. The amount of proteins added to the medium (M) before transduction is shown. Cell lysates (CL) were prepared after 4h of transduction and the internalization of the transduced proteins was visualized by gel electrophoresis and western blotting analysis using a His antibody. Reprobing the blot with anti-tubulin verified equal loading of the gel.
   **Figure 6** C) depicts a Western blot analysis of Stat3 and phosphorylated Stat3 (P-Stat3) in cells grown in the presence or absence of serum (10% FCS) confirming constitutive activation of Stat3 in MZ-54 glioblastoma cells. As a control for inducible Stat3 phosphorylation, HepG2 liver carcinoma cells were induced with IL-6 (10 ng/ml).
   **Figures 6D-E)** depict viability. The viability of NIH3T3 and MZ54 cells treated with the indicated aptamers (1 µM) was determined with an XTT assay. Transduction was performed in DMEM medium containing 2% FCS. The medium and peptides were replaced in 24h intervals. Viability was measured daily. The assay was performed in duplicated and repeated twice with independently prepared batches of protein. Shown are the results obtained from one typical experiment.
**Figure 7****:**

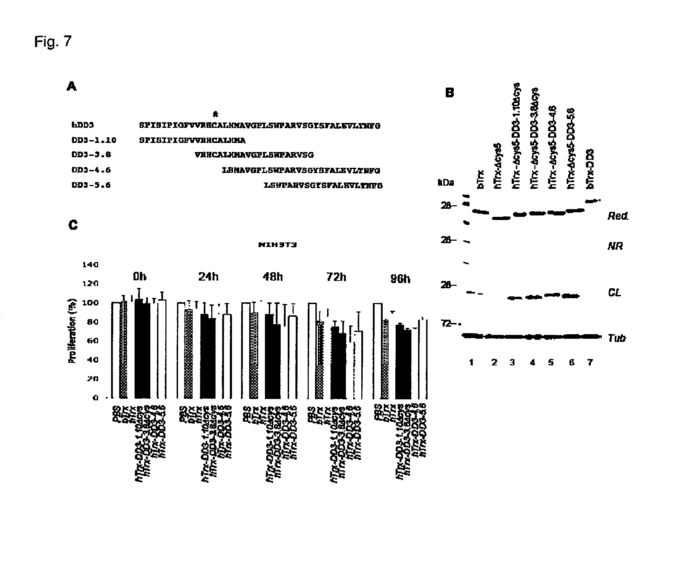

   **Figure 7A****)** depicts the delimitation of the Stat3 binding DD3 aptamer sequence and assay of its transduction properties. The DD3 aptamer sequence comprises 40 amino acids. Four overlapping subfragments (DD3-1.10, DD3-3.8, DD3-4.6 and DD3-5.6) were derived and cloned into the hTrx-_Δcys5 scaffold protein. Amino acids added to the sequence by the cloning procedure are shown in grey. The cysteine shown in bold and marked by an asterisk was replaced with a serine residue.
   **Figure 7B****)** depicts a western blot. Purified bTrx and hTrx based aptamer fusion proteins (1 µg each) were analyzed under reducing (Red.) and non-reducing conditions (NR) in a coomassie gel. The proteins were transduced into NIH3T3 cells (1 µM each) and cell lysates (CL) were obtained 4 h after transduction. Uptake of the aptamers was monitored in a western blot using a His-tag antibody. -tubulin served as loading control.
   **Figure 7C****)** depicts a cell vuability assay. Cell viability assay (XTT) of NIH3T3 cells treated with the indicated aptamers (1 µM). Conditions are described in Fig. 6D-E. At every time point the viability of the cells treated with PBS was determined and set to 100% and compared to the viability data of the protein treated cells. Shown are the mean values of three independent measurements using different protein batches.
**Figure 8****:**

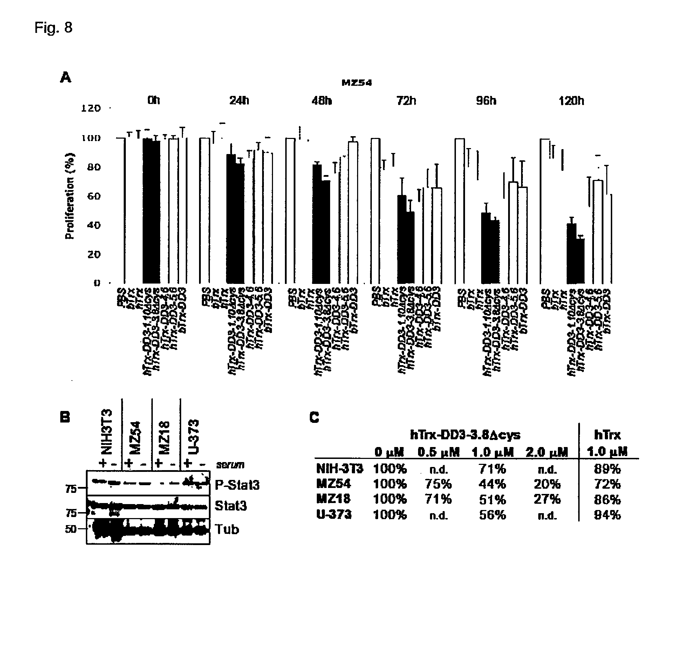

   **Figure 8A****)** MZ54 cells were treated with 1 µM of the peptide aptamers indicated in A and their viability was determined in an XTT assay. The medium (DMEM with 2% FCS) with the peptides was replaced once a day. At every time point the viability of the cells treated with PBS was determined and set to 100% and compared to the viability of the protein treated cells. Mean values represent data obtained with three independent measurements using different batches of protein.
   **Figure 8B****)** Western blot analysis of Stat3 phosphorylation in glioblastoma cells and NIH3T3 control cells. To confirm constitutive activation of Stat3 in two other malignant glioblastoma cell lines cells, cell lysates of MZ18 and U-373 cells grown in the presence of absence of 10 % FCS were prepared in addition to MZ54 and NIH3T3 lysates. Blots prepared from the lysates were incubated with a phospho-Stat3 (Tyr705) specific antibody or with anti-Stat3 to detect the total level of Stat3 proteins in the lysates. As a loading control, blots were incubated with an anti- - tubulin antibody.
   **Figure 8C****)** The cells mentioned for Figure 8B) were treated with PBS (0 µM peptide aptamer) or increasing concentrations (0.5, 1 or 2 µM) of peptide aptamer hTrx-DD3-3.8Δcys or the empty scaffold (hTrx-Dcys5). After 96 h proliferation of the cells was measured in an XTT assay. The proliferation rate of the PBS treated cells was set to 100% for each cell line and compared to the peptide aptamer treated cells (n.d.; not determined).
**Figure 9****:**

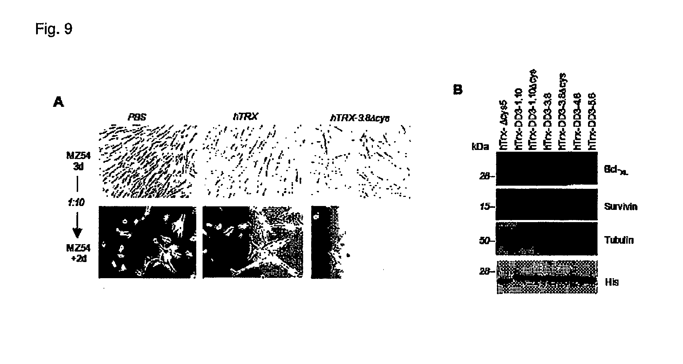

   **Figure 9 A)** depicts the cellular morphology of MZ54 cells treated with Stat3 specific peptide aptamer hTrx-DD3.8_Δcys. Cells were grown in DMEM with 2% FCS and treated for 3 days with PBS, the empty scaffold (hTrx-Δcys5, 1 µM) or with the aptamer (hTrx-DD3.8-Δcys, 1 µM) (upper row). Cells were trypsinized and seeded onto new plates (1:10 dilution). After 2 days the cells were photographed (lower row).
   **Figure 9B****)** depicts a western blot analysis of cells treated for 4h with the indicated peptide aptamers (1 µM) or with the empty scaffold protein (hTrx-Dcys5). Total cellular proteins were prepared and analyzed by gel electrophoresis and western blotting. To confirm uptake of the peptide aptamers, the cell lysates were probed with a His-Tag antibody (Qiagen). To detect induction of apoptosis by down-regulation of anti-apoptotic genes, blots were incubated with Bcl-x (PharMingen) or surviving (Novus Biologicals) antibodies. The detection of β-tubulin (Sigma) served as a loading control.

### Examples:

The following examples are intended to illustrate the invention further. They are not intended to limit the subject matter of the invention thereto.

### General Materials and Methods

### Cell Lines and Cell Culture

Cells were maintained in DMEM containing 10% FCS and 4 mmol/L glutamine. SKBR3 cells were used for isolation of the ErbB2 receptor for coimmunoprecipitation analysis. NIH3T3 murine embryonic fibroblasts and the glioblastoma cell lines MZ54, MZ18 and U-373 were used for XTT analysis to determine the effects of peptide aptamers on cell viability.

### Construction of Plasmids

For interaction assays the yeast-two-hybrid system pGAD-T7 and pGBKT7 vectors (Clontech) were used. To fuse the scaffolds with the Gal4-AD, they were cloned between the *Nde* I and *Xho* I restriction sites. For bacterial expression, the pET30 vector (Merck) was used. The MCS was removed and the scaffolds were cloned in the *Bg*/ *II*/*EcoR* I site directly behind the S-tag. After the last scaffold codon, the PTD was cloned using the *EcoR* I and *Xho* I restriction sites and obtained a second His-tag at the 3' end. To clone the CCSL scaffold two complementary oligonucleotides were designed with the following sequence: 5'-GGGGAATTCCATATGAAGCTTTACGCGGCTCTGGAGAGCGAAGTAAGTGCGCTCG AATCCGAAGTCGCGAGTCTCGAAAGCGAAGGCGCCGGTGGAGGCGGTCCGGGT-3'. After annealing the two oligonucleotides they were cut with *RsrII* and ligated to a second DNA fragment comprised of two annealed complimentary oligonucleotides with the following sequence: 5'-CAGCGGACCGGGTGGTGGGCCAAAAGCAAACTCTCTGCGGTCAAATCCAAACTCGC GAGTGTAAAATCCAAACTCGCTGCGTACGAATTCTCTAGA-3'. The complete fragment was cloned into pET30. Between the coils at the *Rsr*II restriction site a linker sequence (5'-CGGTCCGGGTTTGGCCCATGGTTATGTTAACGGGGCTCGAGGTGGCGGTCCG-3') was inserted mimicking a sequence for a 12mer aptamer. Two restriction sites were included for directional cloning of randomized sequences. For expression and purification of CCSL scaffold of yeast cells, the sequence was also inserted in the *Sna*B I and *Eco*R I site of the PIC9 vector (Invitrogen). To clone the DD domains a nucleotide with the sequence: 5'-AAAGATCTGCATATGTAGGTAGGTTCTAAAGTTATTCTGTTfGAAGGCGGTGGCGGC CATGGTGGGGCTCGAGGCGGTCCGGGTGGTTCTAAAGTTATTCTGTTTGAAGGTGAATT CGTCTAGAAAGGGCCCAAA3' was amplified with two flanking primers containing the Nde I and EcoR I restriction sites. The oligonucleotide already contained a linker region flanked by two Rsr II sites for cloning of the randomized aptamer sequences.

The hTrx scaffold sequence was amplified from cDNA prepared from human cells by using four primers:
F1: 5'-CCAGATCTGATGGTGAAGCAGATCGAG-3',
F2: 5-' *GGC*GGTCCG*GGC*AAAATGATCAAGCCTTTC-3',
R1: 5'-*GCC*CGGACC*GCC*CCACGTGGCTGAGAA-3',
R2: 5'-GAATTCGACTAATTCATTAATGGT-3'.

Two fragments of hTrx were amplified and the sequence in the active site was replaced with an Rsr II site for cloning of aptamers. The cysteins encoded at this site were replaced with glycins (italics). To mutate the cysteins in the C-terminal of hTrx site directed mutagenesis was applied using the QuickChange II kit according to the manufacturer's protocol (Stratagene, Amsterdam, The Netherlands).

Cloning of the aptamers (DBD1: PLTAVFWLIYVLAKALVTVC, DD3: SPISIPIGFVVRHCALHMAVGPLSWPARVSGYSFALEVLTNF, All-7: PLNFYRHGFLPNAVMASMLEVGPWFELLGPCGLAGHPLSSLRI) in hTrx was performed using the *Rsr*II/*Ava* II restriction sites flanking the aptamer in bTrx and ligating it in the *Rsr*II site of hTrx.

The construction of the bTrx expression plasmids (pFlag-bTrx and pET-bTrx) is described above. Mutagenesis of the cysteins in the active site of bTrx was also performed with the QuickChange II kit (Stratagene, Amsterdam, The Netherlands)

### Bacterial Expression and Purification of Peptide Aptamers

Constructs were transformed in BL21-codon+ cells and grown overnight until the OD600 reached 0.6. Peptide aptamer expression was induced with 1 mmol/L isopropyl-1-thio- -D-galactopyranoside for 4 h at 30°C. Cells were harvested and proteins were purified under denaturing conditions using urea buffer (8 M urea, 500 mM NaCl in PBS, pH7.5) on an FPLC system (GE Healthcare). Elution of the proteins from the nickel column was performend with an 80-300 mM imidazole (in urea buffer) gradient. Samples were dialysed and refolded in 125 volumes dialysis buffer (400 mM Arginine-HCl, 10% glycerol, 500 mM NaCl in PBS, pH 7.5) overnight. Then, ¼ buffer volume was replaced with 1 x PBS/10% glycerol every 2 h (4 times). Proteins were stored in small aliquots at -80°C.

### Protein Transduction of Purified Peptide Aptamers

To transduce eukaryotic cells (NIH3T3, MZ54), cells were plated in a 6-well plate and grown overnight in DMEM. Before transduction, the concentration of the peptides was adjusted to 10 µM in 100 µL PBS. Proteins were added to 900 µL medium (DMEM / 2%FCS) at a final concentration of 1 µM. Peptides were incubated with the cells for at least 4 h or up to 24 h. For long-term experiments, medium with the peptides was refreshed every 24 h. To analyze uptake of the peptides in the cells a Western blot analysis was performed.

### Western Blot Analysis

Cells were washed two times with ice cold PBS and to remove peptides from the cell surface additionally with acid (0.2 mmol/L acidic acid) on ice, solubilized in RIPA lysis buffer (50 mmol/L Tris (pH7,4), 150 mmol/L NaCl, 1 % NP40, 0.5% sodium desoxicholate, 1 mmol/L EDTA, protease inhibitiors) and incubated on ice for 20 min. Lysates were clarified by centrifugation at 16,000 x g for 10 min. For SDS-PAGE 20 µg of each protein sample was loaded. Gels were blotted onto nitrocellulose membranes, which were probed with specific antibodies as indicated in the figure legends. Proteins were visualized with peroxidase-coupled secondary antibodies using the chemiluminiscence system (GE Healthcare).

### Immunofluorescence Imaging

Cells were grown on coverslips and after transduction peptide aptamers untransduced proteins were removed by washing two times with 3 ml PBS and once with acid for 5 min at 4°C. Cells were fixed with ice cold methanol and permeabilized with 0.1% Tween 20. After blocking the cells, they were incubated with primary antibodies at 4°C overnight. After extensive washing fluorescently labeled antibodies (Molecular Probes, Karlsruhe, Germany) were incubated with the cells for 1 h at room temperature in the dark. The stained proteins were visualized with a confocal laser scanning microscope (Leica, Bensheim, Germany). To stain the nuclei DAPI at a concentration of 1 mg/mL was added in the mounting medium (Antifade, Molecular Probes).

### Immunoprecipitation Analysis

To co-precipitate the ErbB2 receptor, 1 mg protein of SKBR3 cell lysates were incubated with 0.5 mg purified peptide aptamers, 2 µg anti-ErbB2 antibody and 30 µL Protein A beads (Dynal Biotech) for 1-2 h at 4°C. The beads were washed 3x with 1 ml NP40-binding buffer for 5 min at 4°C. Bound proteins were eluted from the beads by adding RotiLoad buffer (Roth) and heating the sample at 95°C for 3 min. To detect the bound aptamers Western blots were prepared and aptamers were detected using a Flag antibody (Sigma).

### Proliferation and Viability Assay

XTT assays were performed to study cell viability after peptide aptamer treatment. 2000 cells/ 100 µL medium (DMEM) were seeded in each well of a 96-well plate. The next day the medium was removed and peptide aptamers dissolved in 10 µL PBS were added to 100 µL medium (DMEM with 2% FCS) to obtain a final concentration of 1 µM. Treatment with aptamers was repeated every day. XTT measurements were performed according to the manufacturer's protocol (Roche Molecular Biochemicals), which assesses cell viability via bioreduction of a tetrazolium compound. Absorbance was measured 4 h after addition of the substrate at 490 nm in a plate reader.

### Example 1:

**Purification properties of the bacterial thioredoxin (bTrx) and its effect on the protein transduction efficiency into target cells**

Purification and transduction properties of peptide aptamers embedded in the bTrx scaffold were investigated by carrying out experiments with a single peptide aptamer clone. The sequence was chosen randomly and the peptide aptamer was not selected for its binding ability to a particular target. The bTrx-peptide aptamer sequence construct was cloned into the pET30 vector (Novagene). Concretely the fusion proteins are encoded by two different vectors pET30 (Novagen) with a T7 promoter and pFlag (Sigma, Taufkirchen, Germany) with a tac promoter for IPTG inducible expression of the recombinant proteins. The Flag-tag served for specific antibody recognition in immuno florescence experiments. The scaffold was also fused to one or two His-tags for purification by nickel chromatography and to a protein transduction domain (PTD) consisting of nine arginines (9R-PTD) (Fig. 1A). There the position of the aptamer between the two cysteine residues is indicated.

Bacterial thioredoxin without (bTrx) and with an aptamer insert (bTrx-Apta) were expressed in bacteria and lysates were obtained. The recombinant proteins were purified by nickel column chromatography and 2 µg were separated by gel electrophoresis under reducing and non-reducing conditions. The proteins were visualized by staining with Coomassie blue. The gel electrophoretic analysis of the proteins purified by nickelchromatography under non-reducing conditions showed that the bTrx-peptide aptamer fusion protein (bTrx-Apta) is present mainly as an oligomer of high molecular weight (Fig. 1B, lane 4). To a lesser extent, this was also true for the bTrx protein without a peptide aptamer insert (Fig. 1B, lane 3). The establishment of reducing conditions by addition of -mercaptoethanol to the loading buffer caused disaggregation of the complexes and the appearance of non-covalently linked proteins. This indicates that the aggregates are formed through disulfide bridges (Fig. 1B, lanes 1 and 2). For other aptamer sequence inserts the same phenomenon was observed.

In addition these results were compared to an expression system in which the recombinant protein is regulated by a tac promoter (pFlag-bTrx-A, Fig. 1A). The expression of the recombinant proteins encoded in the pFlag vector is also induced with IPTG, but the expression rate is lower, thereby increasing the time for protein refolding and possibly resulting in a reduced amount of missfolded proteins. The proteins were also expressed at lower temperatures. Gel electrophoretic analysis of the purified proteins showed that the exchange of the bacterial promoter, the alterations in the culture conditions, or variations in the protein purification protocols did not influence the tendency of the proteins to aggregate.

To test the prerequisite for the inhibition of cellular signaling pathways, the ability of recombinant peptide aptamers to enter cells, bTrx aptamer proteins were fused with a PTD and the ability of the purified proteins shown in Fig. 1B to penetrate cells was investigated. The purified proteins were added to the medium of cultured cells (NIH3T3). After 4 h the cells exposed to the purified bTrx or the bTrx-Apta proteins were washed with PBS, or with PBS and acid (0.2 M acetic acid in PBS). The acid wash efficiently removes proteins/aptamers, which merely adhere to the cell surface. Whole cell extracts were prepared, the proteins (20 µg) were separated by gel electrophoresis and the presence of the recombinant proteins, internalized bTrx or bTrx-Apta fusion proteins, was monitored in Western blots (Fig. 1C) with an antibody specific for the His-tag. Uptake of bTrx, but not of bTrx-Apta, was also detected with a His-tag specific antibody (Fig. 1C, lanes 1-3 and lanes 4-6). The negative control represents lysates of non-transduced cells. The detection of actin served as a loading control.

To investigate why the bTrx-Apta proteins were not detected in the cell lysates, fluorescence microscopy was performed to visualize the proteins after transduction (Fig. 1D). After transduction of NIH3T3 cells with bTrx and bTrx-Apta for 4 h, the cells were fixed and stained with an anti-Flag antibody to detect the recombinant proteins. Nuclei were stained with DAPI. PC; phase contrast imaging.The bTrx proteins showed an even distribution within the cells. In contrast, bTrx-Apta proteins were not detected intracellularly, but were found in aggregates.

This observation was further analyzed using confocal laser scanning microscopy (Fig. 1E). Cells were transduced for 4 h with bTrx (left) or with peptide aptamer bTrx-AII-7 (middle and right panel). After fixation of the cells a Flag antibody was used for detection of the proteins using a standard immunofluorescence protocol. The aggregated form of the bTrx-aptamer protein was thus unable to undergo PTD mediated cellular uptake and merely adhered to the cell membrane on the surface of the cells.

### Example 2:

**The influence of cysteines in the bTrx scaffold on aggregation, uptake and binding efficiency of the recombinant proteins**

A recombinant bTrx construct, into which the aptamer II-7 (All-7) was inserted, was expressed and purified. This aptamer specifically binds to the intracellular domain of the ErbB2 receptor. When recombinant bTrx-AII-7 was analyzed by gel electrophoresis in the presence of 1 mM DTT under reducing conditions, the high molecular weight aggregates (Fig.2A, lane 1-2) were strongly diminished and the molecule migrated mainly as a monomer (Fig. 2A, lanes 5-6), thus hinting at a role of an intramolecular disulfide bond formation. Similar observations were made when 0,3 M DTT was added directly after purification for 2h (Fig. 2A, lanes 3,4,7,8). In this case DTT was slowly removed during subsequent dialysis steps, allowing correct refolding of the proteins. After this treatment most of the proteins appeared to be non-covalently linked. Only a small amount of dimers was detected (Fig. 2A, lanes 3 and 4).

BTrx contains two cysteines flanking the active site in which the aptamer is inserted (Fig. 1A). Theses cysteines in the active site of the bTrx protein were replaced with serine residues. The removal of these cysteines resulted in a strong reduction of the high molecular weight material detected upon protein purification (Fig. 2A, lanes 10 and 12) when compared to the wild type proteins (lanes 9 and 11). The mutated protein is largely present as a monomer, similar to the cysteine containing proteins obtained upon DTT treatment (Fig. 2A, lanes 3 and 4).

It was further investigated, if replacement of the two cysteines in the bTrx scaffold by serines affects its functional properties, i.e. the binding to its target structure and its cellular uptake. Recognition of the ErbB2 receptor by the All-7 variants was tested in coimmunoprecipitation (co-IP) experiments. Membrane extracts from SK-BR3 cells, enriched in the ErbB2 receptor, were mixed with the AII-7 aptamer in wild-type bTrx (wt) or mutated bTrx (mt) and the ensuing protein complexes were immunoprecipitated with an ErbB2 specific antibody. Equal amounts (Fig.2B, lane1-3) of wt bTrx-AII-7 and mt bTrx-AII-7 were added to the ErbB2 containing cell extracts and the amount of AII-7 aptamer present in the immunoprecipitates was visualized by Western blotting (Fig. 2B, lane 4-6). The co-immunoprecipitations showed that the exchange of the cysteine residues by serines enhanced recognition and resulted in a 2.4-fold increase in binding to the target protein (Fig. 2B, compare lanes 5 and 6).

Also the cellular uptake and the intracellular localization of the wt bTrx-AII-7 and the mt bTrx-AII-7 proteins were analyzed. Immunofluorescence microscopy showed that the aptamers inserted into the mutated bTrx scaffold are able to enter the cells efficiently and co-localize intracellularly with the ErbB2 receptor (Fig. 2C). The protein aggregates associated with the cell surface - observed previously - are not present. The homogenous staining of the All-7 aptamers throughout the cytoplasm also attests the efficient uptake. The colocalization (light) of the All-7 and the ErbB2 receptor suggests intracellular target recognition.

Accordingly, the biological properties of the peptide aptamers are strongly dependent upon the configuration in which they are present upon expression and purification. Oligomerization very much impedes their binding and cellular uptake potential. This is mainly caused by the two cysteines in the active site, which flank the inserted aptamer sequences. This effect is especially strong when aptamers of 40 amino acids are inserted into the active site, but was also found regularly in constructs with inserts of 20 amino acids.

### Example 3:

### Optimization of the human thioredoxin molecule as a scaffold structure

Following the results from example 2 cysteine residues were also removed from human thioredoxin (hTrx). Human thioredoxin (hTrx) is a protein of 105 amino acids. The overall structure of the human protein is conserved when compared to its bacterial counterpart (Fig. 3A). Two cysteines residues are present at the active site of the hTrx, similar to the ones observed in the bTrx molecule. These two cysteines (cys32 and cys35) were removed and replaced by two glycines to prevent intermolecular multimerization of the recombinantly expressed proteins. Glycine residues provide for flexibility of the inserted peptide aptamers and allow them to adopt their own conformation. Additionally, an *Rsr*II restriction site for the cloning of a linker sequence by random mutagenesis (Fig. 3A) was introduced. This scaffold protein (hTrx-Δcys2) can be highly expressed in bacteria. The insertion of various peptide aptamers in hTrx usually resulted in about 2-fold higher yields when compared to the same peptide aptamers inserted into bTrx. This scaffold construct was purified under denaturing conditions in good quantities. About 10 mg of hTrx-Δcys2 protein per 1 L culture were obtained as compared to 6 mg/ L culture for the bTrx construct. The replacement of the two cysteine residues by glycines greatly improved the yields in the purification of this scaffold. Soluble proteins were obtained, which migrated in a single band in gel electrophoretic analysis under non-reducing conditions (Fig. 3B). Only a small fraction of the proteins still precipitated during dialysis.

Under the assumption that three additional cysteine residues at position 62, 69 and 73 in the carboxyl terminal part of the protein might be the cause. Cys62,cys69 and cys73 were replaced by serines by site directed mutagenesis generating hTrx-Δcys5 (Fig. 3A). This further improved the yields of the purified hTrx recombinant scaffold proteins to about 14 mg/L of culture (Fig. 3B). Gel filtration experiments were carried out and confirmed the monomeric nature of the purified proteins lacking the five cysteine residues.

The hTrx scaffold, devoid of cysteines, was fused to the Gal4-AD resulting in a construct that can be used for the construction of peptide aptamer libraries and yeast-two-hybrid screening experiments (Fig. 3C). In order to be able to insert a randomized peptide aptamer library unidirectionally, the restriction sites *Bam*H I and *Xma* I were added to the linker. The sequence of the linker in the active site is shown in Fig. 3C. The selected restriction sites encode additional glycine and proline residues. The additional glycines might give the aptamer structure more flexibility. Proline forms protrusions to the outside of a protein and might be advantageous for the presentation of peptide aptamers in the context of the scaffold. The formation of the protrusions in the hTrxΔcys5-L scaffold was analyzed by determining the accessibility of the insertion site using the ProtScale program (30).

It was found that the insertion of the prolines indeed enhances the formation of a protrusion at the active site when compared to the wild-type hTrx molecule (Fig. 3D).

The expression of the hTrxDcys5-L as a Gal4-AD fusion protein in yeast cells was confirmed. So, the alterations in the hTrx molecule were compatible with its scaffold properties and the presentation of inserted peptide aptamers. The high expression levels and the good purification properties of this scaffold protein are favorable vantage points for testing selected peptide aptamers for therapeutic purposes.

### Example 4:

### Incorporation of previously identified target specific peptide aptamers into the optimized hTrx scaffold

It was tested if the peptide aptamers originally identified as bTrx-peptide aptamers can be transferred into the optimized hTrx scaffold according to example 3.

Three peptide aptamers isolated from our initial library screen were cloned into the hTrx scaffold: (i) peptide aptamer DD3 with binding specificity for the dimerization domain of Stat3; (ii) peptide aptamer DBD1 with binding specificity to the DNA-binding domain of Stat3; both peptide aptamers are able to disrupt Stat3-dependent transcriptional transactivation and (iii) peptide aptamer All-7 with binding specificity to the kinase domain of the ErbB2 receptor and the ability to interfere with Akt kinase activation. The DD3 and All-7 aptamers are 40 amino acids in length; two 20 amino acid sequences were inserted in tandem during the construction of the library. Aptamer DBD1 is 20 amino acids in length.

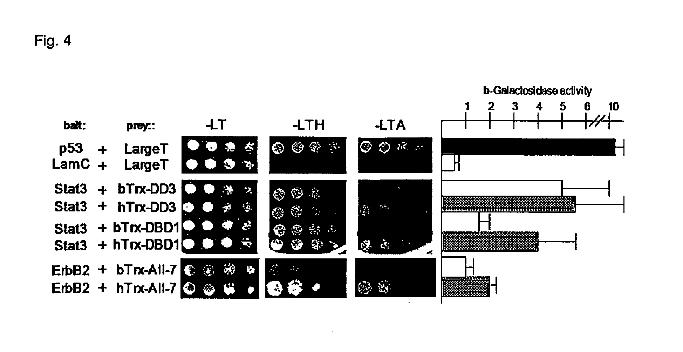

The yeast-two-hybrid system was used to verify binding of the peptide aptamer constructs to their target domains (Fig. 4). If the interactions between the target protein (bait protein fused to the Gal-4 DNA binding domain) and the peptide aptamer (prey protein fused to Gal4-TAD) occur, the AH109 yeast cells are able to grow on selective medium lacking histidine (-LTH). If the interaction is strong enough, the cells are able to grow on medium lacking adenine (-LTA). The insertion of aptamer DD3 into hTrx did not change the ability of the yeast cells to grow on -LTA medium when compared to cells, which expressed DD3 in the context of bTrx. In -galactosidase assays a slight increase in activity was observed indicating better binding properties. For aptamer DBD1, the interaction with Stat3 is relatively weak, because only growth on -LTH plates was observed. However, if this aptamer sequence was inserted into hTrx, growth of colonies on -LTA plates was clearly visible again showing improved binding. Also the -galactosidase assays showed a significant increase in activity. For aptamer All-7 also an increase in its ability to bind to ErbB2 upon insertion in the hTrx scaffold was observed.

Thus, the peptide aptamers known in the context of bTrx can be transferred to the modified hTrx scaffold and presentation of these aptamers in this scaffold even enhances binding to their targets.

### Example 5:

### Protein transduction properties of the hTrx scaffold

The application of peptide aptamers as inhibitors of signal transduction events and their use in a therapeutic fashion is crucially dependent upon their uptake into cells. This process can be mediated by a protein transduction domain, PTD. A short sequence derived from the HIV-TAT-protein is widely used for this purpose, but a sequence of 9 arginine residues (9R) was reported to be even more effective (31). Other reports have shown that transduction depends on cargo or cell type used.

Increasing amounts of the purified proteins fused to 9R were added to the culture medium of different cells (e.g. NiH3T3, 293T, HepG2, MCF7). By comparing fluorescence intensities, it was found that the hTrx scaffold in which all cysteines had been mutated to serines or glycines was more efficiently taken up by cells than the wild type hTrx scaffold (Fig. 5A). Also the characteristics of a TAT-PTD construct was compared with those of a 9R-PTD construct. The hTrx scaffold was fused with the TAT-PTD at the carboxyl terminus and the purification and cellular uptake properties of the proteins were investigated. Generally, the yield of recombinantly expressed hTrx-9R was higher than that of hTrx-TAT upon purification. The hTrx-TAT protein also yielded two bands in the gel electrophoretic analysis after purification (Fig. 5B, lane 5). The yield of both proteins as monomers was improved after deletion of the cysteines in the scaffold. Higher amounts were obtained and only monomers were observed under non-reducing conditions (Fig.5B, lane 6-9).

The efficiency of protein transduction for the proteins comprising the different PTDs were compared and the hTrx fusion proteins were transcuced into the cell types described above. 4 hours after addition of the proteins in the medium, cell lysates were prepared (Fig. 5C). Both constructs, hTrx-9R and hTrx-TAT, were taken up by the cells with similar efficiencies (Fig. 5C, showing for NIH3T3 cells). Taking the results of Fig. 5B and Fig. 5C into account, the hTrxΔcys5 scaffold was fused to the 9R-PTD.

### Example 6:

### Enhanced intracellular inhibition of Stat3 signaling by peptide aptamers integrated into the hTrx scaffold devoid of cysteines

The peptide aptamers DD3 and DBD1 have binding specificities for the transcription factor Stat3 and show effects on the proliferation of cancer cells (Nagel-Wolfrum et al. Mol Cancer Res 2004;2(3):170-82.) Fig. 6A shows the recombinant proteins bTrx DBD1 and bTrx-DD3 upon purification. Although a fair amount of protein could be isolated (lanes 3 and 4), only a small fraction is present as monomeric proteins under non-reducing conditions (lanes 6 and 7). Multimerisation does not seem to affect the native bTrx molecule without a peptide insert (lanes 2 and 5).

Multimerisation is also the major impediment for peptide aptamer uptake into cultured cells. Two established cell lines, NIH3T3 mouse fibroblasts and MZ-54 glioblastoma cells, which were derived from a humanpatient with a grade IV astrocytoma (44), were transduced with different peptide aptamer constructs and monitored the uptake of these molecules into the cytoplasm of the cells (Fig. 6B). Although equal amounts were added in the medium (lanes 1-3) very little bTrx-DBD or bTrx-DD3 could be detected in the lysates of both cell lines (lanes 6 and 7). Efficient uptake of bTrx, the molecule not affected by aggregation, was found (lane 5). NIH3T3 cells express Stat3, but growth and survival of NIH3T3 cells is not dependent upon Stat3 activation. The XTT proliferation assays confirmed that the addition of the bTrx peptide aptamers into the medium of these cells did not affect their growth kinetics (Fig. 6D). MZ-54 glioblastoma cells express constitutively activated Stat3 (Fig. 6C) as compared to HepG2 cells, in which Stat3 phosphorylation is inducible upon addition of IL-6. MZ54 cells are dependent upon Stat3 function. Only a modest effect on MZ-54 growth can be seen in XTT assays upon treatment of the cells over 5 days with 1 µM of the poorly internalizing peptide aptamers bTrx-DBD and bTrx-DD3 (Fig. 6E).

It was investigated, if the improved monomer yields and uptake of aptamers inserted into the hTrx scaffold would translate into enhanced inhibition effects. The DD3 aptamer was used for these experiments, since it binds to Stat3 with high affinity. The aptamer insert was originally 40 amino acids in length. Since this is a relatively large aptamer, which might destabilize the protein, the deletion experiments were carried out to reduce it to 20 amino acids. The specific binding abilities of the shortened peptides was monitored in yeast two-hybrid-assays.

The fragments covering DD3 (1.10, 3.8, 4.6 and 5.6) shown in Fig. 7A were cloned into a bacterial expression vector and the different hTrx-peptide aptamers fused to a 9R-PTD were purified (Fig. 7B). Furthermore, the cysteine residue present in the DD3 aptamer was mutated to serine to prevent dimerization of purified proteins, which was still observed upon gel electrophoresis under nonreducing conditions (not shown). The resulting peptide aptamers, hTrxΔcys5-DD3-1.10Δcys and hTrxΔcys5-DD3-3.8Δcys, shown in 7B, lane 4 and 5, migrated only as monomers in the gel electrophoretic analysis under reducing and non-reducing conditions. These proteins were used to transduce NIH3T3 cells and the uptake of the peptide aptamers into the cytoplasm (CL) was monitored (Fig. 7B). The blot confirmed the improved uptake of the DD3 fragments in hTrx (lane 4-7) compared to the original bTrx-DD3 peptide aptamer (lane 8). After confirming the uptake of the peptides in the NIH3T3 cells, an XTT assay was performed to analyze the effects of the peptides on the viability of these cells. Since the proliferation of NIH3T3 cells is not dependent on Stat3 signaling, cells transduced with the peptide aptamers were not affected in their growth kinetics (Fig. 7C). The different peptide aptamers shown in Fig. 7B were also introduced into the glioma cell line MZ54 and uptake was monitored by Western blotting. XTT assays were carried out to monitor cell viability and PBS treated cells were used as controls. The results show that cells treated with the empty scaffolds bTrx and hTrx, the peptides aptamers hTrxΔcys5-DD3-4.6 and hTrxΔcys5-DD3-5.6 were only very slightly affected (Fig. 8A). Also the full length

DD3 fragment integrated into bTrx was very inefficient in inhibiting proliferation.

On the other hand, hTrxΔcys5-DD3-1.10Δcys and hTrxΔcys5-DD3-3.8Δcys showed very strong anti-proliferative effects (Fig. 8A). This indicates that the amino acids interacting with Stat3 are located in the N-terminal part of aptamer DD3. The cytotoxic effects set in after 48 hours and are very pronounced after 96 and 120 hours.

Two other glioblastoma cell lines, MZ18 and U373, were also treated with peptide aptamers. These cells also express constitutively activated Stat3 at similar levels as MZ54 (Fig. 8B). The peptide aptamers hTrxΔcys5-DD3-1.10Δcys and hTrxΔcys5-DD3-3.8Δcys were able to strongly inhibit the proliferation of MZ18 and U373 cells (Fig. 8C). We also studied the dose dependence of the inhibition effects and added increasing concentrations (0.5 µM, 1 µM and 2 µM) of peptide hTrxΔcys5-DD3-3.8Δcys to these cells. The percentage of viable cells was measured 96 hours after addition of the peptide aptamer and a dose-dependent inhibition of proliferation was observed. The proliferation rates of the cells were compared to non-treated cells or cells treated with the empty scaffold protein (Fig. 8C).

### Example 7:

### Transduction of peptide aptamer hTrx-Δcys5-DD3-3.8Δcys

The effects of these peptides aptamers on the cellular morphology of MZ54 cells was tested. Semi-confluent cells were exposed for three days to PBS, hTrx or hTrx-Δcys5-DD3-3.8Δcys (Fig. 9A). Control cells treated with PBS and hTrx continued to grow and reached confluency after three days. Cells exposed to hTrx-Δcys5-DD3-3.8Δcys appeared much less dense (around 50%), were elongated or tended to round up. After three days of treatment with the peptide aptamer, the cells were trypsinized and a 1:10 dilution of the cells was replated. Two days later the cells were photographed again. The PBS and hTrx treated cells attached and showed a normal morphology. In contrast, most of the hTrx-Δcys5-DD3-3.8Δcys treated cells failed to reattach to the plates. The few cells that were able to attach did not divide further.

To investigate, if this phenotype is caused by the induction of apoptosis, cell extracts of MZ54 cells treated with the different aptamers were analyzed for the expression of anti-apoptotic target genes of Stat3. Consistent with previously published results, western blot analysis showed that the loss in cellular viability is accompanied by the down-regulation of the anti-apoptotic regulators Bcl-xL and survivin (Fig. 9B). This result indicates that the cellular phenotypes induced by the peptide aptamers, growth inhibition and induction of cell death, are consequences of the inhibition of the pro-survival Stat3 target genes, survivin and Bcl-xL.

### Sequence Listing:

In SEQ.ID.Nos. 1 to 5 the amino acid sequence is given in one-letter code and alternatively in the corresponding 3-letter code. The binding sequence in case of doubt is the one-letter code. Underlining highlights either a cysteine (or nucleic acid triplets coding for it) to possibly be replaced or the replacement for the cysteine (or triplet), or underlining highlights the (by peptide bond) inserted aptamer. In SEQ.ID.NOs. 30 to 33 the restriction sites for BamHI and Xmal are shown in bold and italics.

SEQ:ID.-No.1
Amino acid sequence of the unmodified bacterial thioredoxin
(wild-type; wt-bTrx)
1 M S D K I I H L T D D S F D T D
17 V L K A D G A I L V D F W A E W
33 C G P C K M I A P I L D E I A D
49 E Y Q G K L T V A K L N I D Q N
65 P G T A P K Y G I R G I P T L L
81 L F K N G E V A T T K V G A L S
97 K G Q L K E F L D A N L A E F G

1 Met Ser Asp Leu Ile Ile His Leu Thr Asp
11 Asp Ser Phe Asp Thr Asp Val Leu Lys Ala
21 Asp Gly Ala Ile Leu Val Asp Phe Trp Ala
31 Glu Trp Cys Gly Pro Cys Lys Met Ile Ala
41 Pro Ile Leu Asp Glu Ile Ala Asp Glu Tyr
51 Gln Gly Lys Leu Thr Val Ala Lys Leu Asn
61 Ile Asp Gln Asn Pro Gly Thr Ala Pro Lys
71 Tyr Gly Ile Arg Gly Ile Pro Thr Leu Leu
80 Leu Phe Lys Asn Gly Glu Val Ala Thr Thr
91 Lys Val Gly Ala Leu Ser Lys Gly Gln Leu
101 Lys Glu Phe Leu Asp Ala Asn Leu Ala Glu
111 Phe Gly

SEQ:ID.-No.2

Amino acid sequence of a modified bacterial thioredoxin (bTrx-
Δcys2)
1 M S D K I I H L T D D S F D T D
17 V L K A D G A I L V D F W A E W
33 S G P S K M I A P I L D E I A D
49 E Y Q G K L T V A K L N I D Q N
65 P G T A P K Y G I R G I P T L L
81 L F K N G E V A T T K V G A L S

97 K G Q L K E F L D A N L A E F G

1 Met Ser Asp Leu Ile Ile His Leu Thr Asp
11 Asp Ser Phe Asp Thr Asp Val Leu Lys Ala
21 Asp Gly Ala Ile Leu Val Asp Phe Trp Ala
31 Glu Trp Ser Gly Pro Ser Lys Met Ile Ala
41 Pro Ile Leu Asp Glu Ile Ala Asp Glu Tyr
51 Gln Gly Lys Leu Thr Val Ala Lys Leu Asn
61 Ile Asp Gln Asn Pro Gly Thr Ala Pro Lys
71 Tyr Gly Ile Arg Gly Ile Pro Thr Leu Leu
80 Leu Phe Lys Asn Gly Glu Val Ala Thr Thr
91 Lys Val Gly Ala Leu Ser Lys Gly Gln Leu
101 Lys Glu Phe Leu Asp Ala Asn Leu Ala Glu
111 Phe Gly

SEQ.ID.No. 3:
Amino acid sequence of the unmodified human thioredoxin
(wild-type; wt-hTrx)
1 M V K Q I E S K T A F Q E A L D
17 A A G D K L V V V D F S A T W C
33 G P C K M I K P F F H S L S E K
49 Y S N V I F L E V D V D D C Q D
65 V A S E C E V K C M P T F Q F F
81 K K G Q K V G E F S G A N K E K
97 L E A T I N E L V

1 Met Val Lys Gln Ile Glu Ser Lys Thr Ala
11 Phe Gln Glu Ala Leu Asp Ala Ala Gly Asp
21 Lys Leu Val Val Val Asp Phe Ser Ala Thr
31 Trp Cys Gly Pro Cys Lys Met Ile Lys Pro
41 Phe Phe His Ser Leu Ser Glu Lys Tyr Ser
51 Asn Val Ile Phe Leu Glu Val Asp Val Asp
61 Asp Cys Gln Asp Val Ala Ser Glu Cys Glu
71 Val Lys Cys Met Pro Thr Phe Gln Phe Phe
81 Lys Lys Gly Gln Lys Val Gly Glu Phe Ser
91 Gly Ala Asn Lys Glu Lys Leu Glu Ala Thr
101 Ile Asn Glu Leu Val

SEQ.ID.No. 4:
Amino acid sequence of a modified human thioredoxin (hTrx-
Δcys2)
1 M V K Q I E S K T A F Q E A L D
17 A A G D K L V V V D F S A T W G
33 G P G K M I K P F F H S L S E K

49 Y S N V I F L E V D V D D C Q D
65 V A S E C E V K C M P T F Q F F
81 K K G Q K V G E F S G A N K E K
97 L E A T I N E L V

1 Met Val Lys Gln Ile Glu Ser Lys Thr Ala
11 Phe Gln Glu Ala Leu Asp Ala Ala Gly Asp
21 Lys Leu Val Val Val Asp Phe Ser Ala Thr
31 Trp Gly Gly Pro Gly Lys Met Ile Lys Pro
41 Phe Phe His Ser Leu Ser Glu Lys Tyr Ser
51 Asn Val Ile Phe Leu Glu Val Asp Val Asp
61 Asp Cys Gln Asp Val Ala Ser Glu Cys Glu
71 Val Lys Cys Met Pro Thr Phe Gln Phe Phe
81 Lys Lys Gly Gln Lys Val Gly Glu Phe Ser
91 Gly Ala Asn Lys Glu Lys Leu Glu Ala Thr
101 Ile Asn Glu Leu Val

SEQ.ID.No. 5:
Amino acid sequence of a modified human thioredoxin (hTrx-
Δcys5)
1 M V K Q I E S K T A F Q E A L D
17 A A G D K L V V V D F S A T W G
33 G P G K M I K P F F H S L S E K
49 Y S N V I F L E V D V D D S Q D
65 V A S E S E V K S M P T F Q F F
81 K K G Q K V G E F S G A N K E K
97 L E A T I N E L V

1 Met Val Lys Gln Ile Glu Ser Lys Thr Ala
11 Phe Gln Glu Ala Leu Asp Ala Ala Gly Asp
21 Lys Leu Val Val Val Asp Phe Ser Ala Thr
31 Trp Gly Gly Pro Gly Lys Met Ile Lys Pro
41 Phe Phe His Ser Leu Ser Glu Lys Tyr Ser
51 Asn Val Ile Phe Leu Glu Val Asp Val Asp
61 Asp Ser Gln Asp Val Ala Ser Glu Ser Glu
71 Val Lys Ser Met Pro Thr Phe Gln Phe Phe
81 Lys Lys Gly Gln Lys Val Gly Glu Phe Ser
91 Gly Ala Asn Lys Glu Lys Leu Glu Ala Thr
101 Ile Asn Glu Leu Val

SEQ.ID.No. 6:

Amino acid sequence of the modified human aptamer DD3
binding to the dimerization domain of the transcription
factor Stat3 (DD3-Δcys)
S P I S I P I G F V V R H S A
L H M A V G P L S W P A R V S
G Y S F A L E V L T N F G

SEQ.ID.No. 7:
Amino acid sequence of the human aptamer DD3 binding to the
dimerization domain of the transcription factor Stat3 (DD3-
wild type)
S P I S I P I G F V V R H C A
L H M A V G P L S W P A R V S
G Y S F A L E V L T N F G

SEQ.ID.No. 8:
Amino acid sequence of the human aptamer DD3 binding to the
dimerization domain of the transcription factor Stat3 (DD3-
1.10)
S P I S I P I G F V V R H C A
L H M A G

SEQ.ID.No. 9:
Shortened modified amino acid sequence of the human aptamer
DD3 binding to the dimerization domain of the transcription
factor Stat3 (DD3-1.10-Δcys)
S P I S I P I G F V V R H S A
L H M A G

SEQ.ID.No. 10:
Shortened amino acid sequence of the human aptamer DD3
binding to the dimerization domain of the transcription
factor Stat3 (DD3-3.8)
V R H C A L H M A V G P L S W
P A R V S G

SEQ.ID.No. 11:

Shortened modified amino acid sequence of the human aptamer
DD3 binding to the dimerization domain of the transcription
factor Stat3 (DD3-3.8-Δcys)
V R H S A L H M A V G P L S W
P A R V S G

SEQ.ID.No. 12:
Shortened amino acid sequence of the human aptamer DD3
binding to the dimerization domain of the transcription
factor Stat3 (DD3-4.6)
L H M A V G P L S W P A R V S
G Y S F A L E V L T N F G

SEQ.ID.No. 13:
Shortened amino acid sequence of the human aptamer DD3
binding to the dimerization domain of the transcription
factor Stat3 (DD3-4.6)
L S W P A R V S G Y S F A L E
V L T N F G

SEQ:ID.-No.14
Amino acid sequence of a Fusion Protein of an aptamer
(Y2..Y4) in a modified bacterial thioredoxin (bTrx-Δcys2)
M S D K I I H L T D D S F D T
D V L K A D G A I L V D F W A
E W S G P Y3..Y4 P G G P S K M I
A P I L D E I A D E Y Q G K L
T V A K L N I D Q N P G T A P
K Y G I R G I P T L L L F K N
G E V A T T K V G A L S K G Q
L K E F L D A N L A E F G

SEQ.ID.No. 15:
Amino acid sequence of a Fusion Protein of an aptamer
(Y2..Y4) in a modified human thioredoxin (hTrx-Δcys2)

M V K Q I E S K T A F Q E A L
D A A G D K L V V V D F S A T
W G G P Y3..Y4 P G G P G K M I K
P F F H S L S E K Y S N V I F
L E V D V D D C Q D V A S E C
E V K C M P T F Q F F K K G Q
K V G E F S G A N K E K L E A
T I N E L V

SEQ.ID.No. 16:

Amino acid sequence of a Fusion Protein of an aptamer
(Y2..Y4) in a modified human thioredoxin (hTrx-Δcys5)
M V K Q I E S K T A F Q E A L
D A A G D K L V V V D F S A T
W G G P Y3..Y4 P G G P G K M I K
P F F H S L S E K Y S N V I F
L E V D V D D S Q D V A S E S
E V K S M P T F Q F F K K G Q
K V G E F S G A N K E K L E A
T I N E L V

SEQ.ID.No. 17:

Amino acid sequence of a Fusion Protein of the shortened
aptamer DD3-1.10 in a modified human thioredoxin (hTrx-
Δcys5)
M V K Q I E S K T A F Q E A L
D A A G D K L V V V D F S A T
W G G P S P I S I P I G F V V
R H C A L H M A G P G G P G K
M I K P F F H S L S E K Y S N
V I F L E V D V D D S Q D V A
S E S E V K S M P T F Q F F K
K G Q K V G E F S G A N K E K
L E A T I N E L V

SEQ.ID.No. 18:

Amino acid sequence of a Fusion Protein of the shortened and
modified aptamer DD3-1.10-ΔCys in a modified human
thioredoxin(hTrx-Δcys5)
M V K Q I E S K T A F Q E A L
D A A G D K L V V V D F S A T
W G G P S P I S I P I G F V V
R H S A L H M A G P G G P G K
M I K P F F H S L S E K Y S N
V I F L E V D V D D S Q D V A
S E S E V K S M P T F Q F F K
K G Q K V G E F S G A N K E K
L E A T I N E L V

SEQ.ID.No. 19:

Amino acid sequence of a Fusion Protein of the shortened
aptamer DD3-3.8 in a modified human thioredoxin (hTrx-Δcys5)
M V K Q I E S K T A F Q E A L
D A A G D K L V V V D F S A T
W G G P S V R H C A L H M A V
G P L S W P A R V S G P G G P
G K M I K P F F H S L S E K Y
S N V I F L E V D V D D S Q D
V A S E S E V K S M P T F Q F
F K K G Q K V G E F S G A N K
E K L E A T I N E L V

SEQ.ID.No. 20:

Amino acid sequence of a Fusion Protein of the shortened and
modified aptamer DD3-3.8-Δcys in a modified human
thioredoxin (hTrx-Δcys5)
M V K Q I E S K T A F Q E A L
D A A G D K L V V V D F S A T
W G G P S V R H S A L H M A V
G P L S W P A R V S G P G G P
G K M I K P F F H S L S E K Y
S N V I F L E V D V D D S Q D
V A S E S E V K S M P T F Q F
F K K G Q K V G E F S G A N K
E K L E A T I N E L V

SEQ.ID.No. 21:

Amino acid sequence of a Fusion Protein of the shortened
aptamer DD3-4.6 in a modified human thioredoxin (hTrx-Δcys5)
M V K Q I E S K T A F Q E A L
D A A G D K L V V V D F S A T

W G G P S L H M A V G P L S W
P A R V S G Y S F A L E V L T
N F G P G G P G K M I K P F F
H S L S E K Y S N V I F L E V
D V D D S Q D V A S E S E V K
S M P T F Q F F K K G Q K V G
E F S G A N K E K L E A T I N
E L V

SEQ.ID.No. 22:

Amino acid sequence of a Fusion Protein of the shortened
aptamer DD3-5.6 in a modified human thioredoxin (hTrx-Δcys5)
M V K Q I E S K T A F Q E A L
D A A G D K L V V V D F S A T
W G G P S L S W P A R V S G Y
S F A L E V L T N F G P G G P
G K M I K P F F H S L S E K Y
S N V I F L E V D V D D S Q D
V A S E S E V K S M P T F Q F
F K K G Q K V G E F S G A N K
E K L E A T I N E L V

SEQ.ID.No. 23:

DNA sequence of a modified bacterial thioredoxin (bTrx-
Δcys2; coding for SEQ.ID.No.2)
ATG AGC GAT AAA ATT ATT CAC CTG ACT GAC GAC AGT TTT GAC ACG

GAT GTA CTC AAA GCG GAC GGG GCG ATC CTC GTC GAT TTC TGG GCA

GAG TGG AGC GGT CCG AGC AAA ATG ATC GCC CCG ATT CTG GAT GAA

ATC GCT GAC GAA TAT CAG GGC AAA CTG ACC GTT GCA AAA CTG AAC

ATC GAT CAA AAC CCT GGC ACT GCG CCG AAA TAT GGC ATC CGT GGT

ATC CCG ACT CTG CTG CTG TTC AAA AAC GGT GAA GTG GCG ACA ACC

AAA GTG GGT GCA CTG TCT AAA GGT CAG TTG AAA GAG TTC CTC GAC

GCT AAC CTG GCC GAA TTC GGG

SEQ.ID.No. 24:

DNA sequence of a modified human thioredoxin (hTrx-Δcys5;
coding for SEQ.ID.No.5)
ATG GTG AAG CAG ATC GAG AGC AAG ACT GCT TTT CAG GAA GCC TTG
GAC GCT GCA GGT GAT AAA CTT GTA GTA GTT GAC TTC TCA GCC ACG
TGG GGC GGT CCG GGC AAA ATG ATC AAG CCT TTC TTT CAT TCC CTC
TCT GAA AAG TAT TCC AAC GTG ATA TTC CTT GAA GTA GAT GTG GAT

GAC TCT CAG GAT GTT GCT TCA GAG TCT GAA GTC AAA AGC ATG CCA
ACA TTC CAG TTT TTT AAG AAG GGA CAA AAG GTG GGT GAA TTT TCT
GGA GCC AAT AAG GAA AAG CTT GAA GCC ACC ATT AAT GAA TTC GTC

SEQ.ID.No. 25:

DNA sequence of the modified human aptamer DD3 binding to
the dimerization domain of the transcription factor Stat3
(DD3-Δcys; coding for SEQ.ID.No. 6)

TCT CCT ATT AGT ATT CCG ATT GGG TTT GTT GTT CGG CAT TCT GCG
CTT CAT ATG GCG GTG GGT CCG TTG TCT TGG CCG GCG AGG GTT TCT
GGG TAT TCG TTT GCT TTG GAG GTG CTG ACT AAT TTC GGT

SEQ.ID.No. 26:

DNA sequence of the shortened human aptamer DD3 binding to
the dimerization domain of the transcription factor Stat3
(DD3-1.10; coding for SEQ.ID.No. 8)

TCT CCT ATT AGT ATT CCG ATT GGG TTT GTT GTT CGG CAT TGT GCG
CTT CAT ATG GCC

SEQ.ID.No. 27:

DNA sequence of the shortened modified human aptamer DD3
binding to the dimerization domain of the transcription
factor Stat3 (DD3-1.10-Δcys; coding for SEQ.ID.No. 9)

TCT CCT ATT AGT ATT CCG ATT GGG TTT GTT GTT CGG CAT TCT GCG
CTT CAT ATG GCC

SEQ.ID.No. 28:

DNA sequence of the shortened human aptamer DD3-3.8 binding
to the dimerization domain of the transcription factor Stat3
(DD3-3.8; coding for SEQ.ID.No. 10)

GTT CGG CAT TGT GCG CTT CAT ATG GCG GTG GGT CCG TTG TCT TGG
CCG GCG AGG GTT TCT GGC

SEQ.ID.No. 29:

DNA sequence of the shortened modified human aptamer DD3-3.8
binding to the dimerization domain of the transcription
factor Stat3 (DD3-3.8-Δcys; coding for SEQ.ID.No. 11)

GTT CGG CAT TCT GCG CTT CAT ATG GCG GTG GGT CCG TTG TCT TGG
CCG GCG AGG GTT TCT GGC

SEQ.ID.No. 30:

DNA sequence of a fusion protein between modified human
thioredoxin (hTrx-Δcys5) and the shortened aptamer DD3-3.8
together with introduced restiction sites

ATG GTG AAG CAG ATC GAG AGC AAG ACT GCT TTT CAG GAA GCC TTG
GAC GCT GCA GGT GAT AAA CTT GTA GTA GTT GAC TTC TCA GCC ACG
TGG GGC GGT CCG GGA TCC GTT CGG CAT TGT GCG CTT CAT ATG GCG
GTG GGT CCG TTG TCT TGG CCG GCG AGG GTT TCT GGC CCG GGC GGT
CCG GGC AAA ATG ATC AAG CCT TTC TTT CAT TCC CTC TCT GAA AAG
TAT TCC AAC GTG ATA TTC CTT GAA GTA GAT GTG GAT GAC TCT CAG
GAT GTT GCT TCA GAG TCT GAA GTC AAA AGC ATG CCA ACA TTC CAG
TTT TTT AAG AAG GGA CAA AAG GTG GGT GAA TTT TCT GGA GCC AAT
AAG GAA AAG CTT GAA GCC ACC ATT AAT GAA TTC GTC

SEQ.ID.No. 31:

DNA sequence of a fusion protein between modified human
thioredoxin (hTrx-Δcys5) and the shortened and modified
aptamer DD3-3.8-Δcys together with introduced restiction
sites

ATG GTG AAG CAG ATC GAG AGC AAG ACT GCT TTT CAG GAA GCC TTG

GAC GCT GCA GGT GAT AAA CTT GTA GTA GTT GAC TTC TCA GCC ACG

TGG GGC GGT CCG GGA TCC GTT CGG CAT TCT GCG CTT CAT ATG GCG

GTG GGT CCG TTG TCT TGG CCG GCG AGG GTT TCT GGC CCG GGC GGT

CCG GGC AAA ATG ATC AAG CCT TTC TTT CAT TCC CTC TCT GAA AAG

TAT TCC AAC GTG ATA TTC CTT GAA GTA GAT GTG GAT GAC TCT CAG

GAT GTT GCT TCA GAG TCT GAA GTC AAA AGC ATG CCA ACA TTC CAG

TTT TTT AAG AAG GGA CAA AAG GTG GGT GAA TTT TCT GGA GCC AAT

AAG GAA AAG CTT GAA GCC ACC ATT AAT GAA TTC GTC

SEQ.ID.No. 32:

DNA sequence of a fusion protein between modified human
thioredoxin (hTrx-Δcys5) and the shortened aptamer DD3-1.10
together with introduced restiction sites

ATG GTG AAG CAG ATC GAG AGC AAG ACT GCT TTT CAG GAA GCC TTG

GAC GCT GCA GGT GAT AAA CTT GTA GTA GTT GAC TTC TCA GCC ACG

TGG GGC GGT CCG GGA TCC TCT CCT ATT AGT ATT CCG ATT GGG TTT

GTT GTT CGG CAT TGT GCG CTT CAT ATG GCC CCG GGC GGT CCG GGC

AAA ATG ATC AAG CCT TTC TTT CAT TCC CTC TCT GAA AAG TAT TCC

AAC GTG ATA TTC CTT GAA GTA GAT GTG GAT GAC TCT CAG GAT GTT

GCT TCA GAG TCT GAA GTC AAA AGC ATG CCA ACA TTC CAG TTT TTT

AAG AAG GGA CAA AAG GTG GGT GAA TTT TCT GGA GCC AAT AAG GAA

AAG CTT GAA GCC ACC ATT AAT GAA TTC GTC

SEQ.ID.No. 33:

DNA sequence of a fusion protein between modified human
thioredoxin (hTrx-Δcys5) and the shortened and modified
aptamer DD3-1.10-Δcys together with introduced restiction
sites

ATG GTG AAG CAG ATC GAG AGC AAG ACT GCT TTT CAG GAA GCC TTG

GAC GCT GCA GGT GAT AAA CTT GTA GTA GTT GAC TTC TCA GCC ACG

TGG GGC GGT CCG GGA TCC TCT CCT ATT AGT ATT CCG ATT GGG TTT

GTT GTT CGG CAT TCT GCG CTT CAT ATG GCC CCG GGC GGT CCG GGC

AAA ATG ATC AAG CCT TTC TTT CAT TCC CTC TCT GAA AAG TAT TCC

AAC GTG ATA TTC CTT GAA GTA GAT GTG GAT GAC TCT CAG GAT GTT

GCT TCA GAG TCT GAA GTC AAA AGC ATG CCA ACA TTC CAG TTT TTT

AAG AAG GGA CAA AAG GTG GGT GAA TTT TCT GGA GCC AAT AAG GAA

AAG CTT GAA GCC ACC ATT AAT GAA TTC GTC

## Claims

1. Peptide aptamer, **characterized in that** at least one cysteine of the wild type of this peptide aptamer is replaced by a non-sulphur-containing amino acid.

2. Peptide aptamer according to claim 1, **characterized in that** the non-sulphur-containing amino acid is selected from glycine, serine, valine, leucine, isoleucin, alanine, lysine, or threonine, preferably is selected from glycine or serine.

3. Peptide aptamer binding to the transcription factor Stat3 according to any of claims 1 or 2, comprising an amino acid sequence according to SEQ.ID.No. 6.

4. Peptide aptamer binding to the transcription factor Stat3 comprising an amino acid sequence of 15 to 30 amino acids being comprised within the amino acid sequence SEQ.ID.Nos. 6 or 7.

5. Peptide aptamer binding to the transcription factor Stat3 comprising an amino acid sequence identical to or showing a similarity of 85% to the SEQ.ID.Nos. 8, 9, 10, 11, 12 or 13; preferably comprising an amino acid sequence identical to or showing a similarity of 90% to the SEQ.ID.Nos. 8, 9, 10, 11, 12 or 13; more preferably comprises an amino acid sequence identical to or showing a similarity of 95% to the SEQ.ID.Nos. 8, 9, 10, 11, 12 or 13.

6. Peptide aptamer according to claim 3 consisting of the amino acid sequence of SEQ.ID.Nos. 8, 9, 10, 11, 12 or 13; preferably consisting of the amino acid sequence of SEQ.ID.Nos. 8, 9, 10 or 11; more preferably consisting of the amino acid sequence of SEQ.ID.Nos. 10 or 11.

7. Nucleic acid coding for a peptide aptamer according to any of claims 1 to 6.

8. Nucleic acid according to claim 7, **characterized in that** the nucleic acid is coding for a protein/peptide according to SEQ.ID.Nos. 6, 8, 9, 10, 11, 12 or 13, preferably to SEQ.ID. Nos. 8, 9, 10 or 11, most preferably to SEQ.ID.Nos. 10 or 11.

9. Nucleic acid according to claim 7, **characterized in that** the nucleic acid comprises a nucleic acid sequence identical to or showing a similarity of 85% to SEQ.ID.Nos. 25, 26, 27, 28 or 29; preferably is comprising a nucleic acid sequence identical to or showing a similarity of 90% to SEQ.ID.Nos. 25, 26, 27, 28 or 29; more preferably comprises a nucleic acid sequence identical to or showing a similarity of 95% to the SEQ.ID.Nos. 25, 26, 27, 28 or 29; highly preferably consists of a nucleic acid sequence identical to or showing a similarity of 95% to the SEQ.ID.Nos. 25, 26, 27, 28 or 29; most preferably consists of a nucleic acid sequence identical to the SEQ.ID.Nos. 28 or 29.

10. Medicament comprising a peptide aptamer according to any of claims 1 to 6, and/or a nucleic acid according to any of claims 7 to 9; and optionally at least one pharmaceutically acceptable carrier.

11. Use of a peptide aptamer according to any of claims 1 to 6, and/or of a nucleic acid according to claim 7 to 9 for the preparation of a medicament for the treatment or prevention of a disease, of symptoms of a disease or causes for a disease connected to the transcription factor Stat3.

12. Use according to claim 11, **characterized in that** the disease is cancer, an autoimmuno-disease, chronic inflammation, psoriasis, a liver disease, preferably is cancer.

13. Use according to claim 11, **characterized in that** the treatment is suppression of tumor cells, suppression of the metastasing potential of tumor cells, activation of immuno cells interacting with tumor cells, removing the block of differentiation of dendritic celss, or inhibition of Stat3 functions.

14. Method for improving the efficacy and/or purification parameters of a peptide aptamer between a peptide aptamer and a Scaffold Protein, wherein in the Scaffold protein and/or in the peptide aptamer at least one cystein of the wild type is replaced by a non-sulphur-containing amino acid, selected from glycine, serine, valine, leucine, isoleucin, alanine, lysine, or threonine, preferably selected from glycine or serine.

15. Kit comprising a composition comprising a peptide aptamer according to any of claims 1 to 6, a nucleic acid according to any of claims 7 to 9, and/or a medicament according to claim 10 and optionally technical instructions with information on the administration and dosage of the composition and/or the medicament.
